# FASCICULE DE BREVET EUROPEEN

(11) **EP 0 663 907 B1**
(45) Date de publication et mention de la délivrance du brevet: **15.01.1997**
(21) Numéro de dépôt: 93921983.8
(22) Date de dépôt: 04.10.1993
(51) Int. Cl.: C07D 305/14, C07D 263/04, C07D 413/12

(54) **PROCEDE DE PREPARATION DE DERIVES DU TAXANE**
VERFAHREN ZUR HERSTELLUNG VON TAXAN-DERIVATEN
METHOD OF PREPARING TAXANE DERIVATIVES

(30) Priorité: 05.10.1992 FR 9211742
(43) Date de publication de la demande: 26.07.1995
(73) Titulaire: RHONE-POULENC RORER S.A., 92160 Antony (FR)
(72) Inventeur: COMMERCON, Alain, F-94400 Vitry-sur-Seine (FR); DIDIER, Eric, F-75013 Paris (FR); FOUQUE, Elie, F-94100 Saint-Maur-des-Fossés (FR)
(74) Mandataire: Pilard, Jacques
(86) Numéro de dépôt international: FR9300968
(87) Numéro de publication internationale: WO9407878

(56) Documents cités:
- WO-A-92/09589
- WO-A-93/16060

## Description

La présente invention concerne un nouveau procédé de préparation de dérivés du taxane de formule générale : 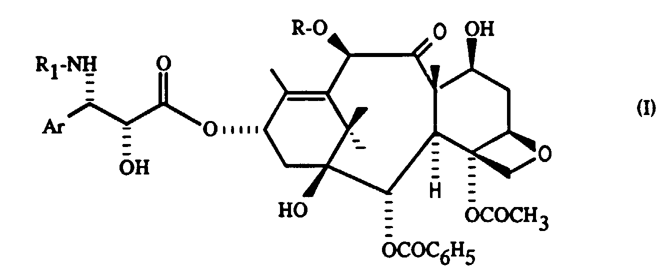 qui présentent des propriétés antileucémiques et antitumorales remarquables.

Dans la formule générale (I):
R représente un atome d'hydrogène ou un radical acétyle, R₁ représente un radical benzoyle ou un radical R₂-O-CO- dans lequel R₂ représente un radical alcoyle, alcényle, alcynyle, cycloalcoyle, cycloalcényle, bicycloalcoyle, phényle ou hétérocyclyle azoté, et Ar représente un radical aryle.

Plus particulièrement, R représente un atome d'hydrogène ou un radical acétyle et R₁ représente un radical benzoyle ou un radical R₂-O-CO- dans lequel R₂ représente:
- un radical alcoyle droit ou ramifié contenant 1 à 8 atomes de carbone, alcényle contenant 2 à 8 atomes de carbone, alcynyle contenant 3 à 8 atomes de carbone, cycloalcoyle contenant 3 à 6 atomes de carbone, cycloalcényle contenant 4 à 6 atomes de carbone ou bicycloalcoyle contenant 7 à 10 atomes de carbone, ces radicaux étant éventuellement substitués par un ou plusieurs substituants choisis parmi les atomes d'halogène et les radicaux hydroxy, alcoyloxy contenant 1 à 4 atomes de carbone, dialcoylamino dont chaque partie alcoyle contient 1 à 4 atomes de carbone, pipéridino, morpholino, pipérazinyl-1 (éventuellement substitué en -4 par un radical alcoyle contenant 1 à 4 atomes de carbone ou par un radical phénylalcoyle dont la partie alcoyl contient 1 à 4 atomes de carbone), cycloalcoyle contenant 3 à 6 atomes de carbone, cycloalcényle contenant 4 à 6 atomes de carbone, phényle, cyano, carboxy ou alcoyloxycarbonyle dont la partie alcoyle contient 1 à 4 atomes de carbone,
- ou un radical phényle éventuellement substitué par un ou plusieurs atomes ou radicaux choisis parmi les radicaux alcoyles contenant 1 à 4 atomes de carbone ou alcoyloxy contenant 1 à 4 atomes de carbone,
- ou un radical hétérocyclyle azoté saturé ou non saturé contenant 5 ou 6 chaînons éventuellement substitué par un ou plusieurs radicaux alcoyles contenant 1 à 4 atomes de carbone,
étant entendu que les radicaux cycloalcoyles, cycloalcényles ou bicycloalcoyles peuvent être éventuellement substitués par un ou plusieurs radicaux alcoyles contenant 1 à 4 atomes de carbone, et
Ar représente un radical phényle ou α- ou β-naphtyle éventuellement substitué par un ou plusieurs atomes ou radicaux choisis parmi les atomes d'halogène (fluor, chlore, brome, iode) et les radicaux alcoyles, alcényles, alcynyles, aryles, arylalcoyles, alcoxy, alcoylthio, aryloxy, arylthio, hydroxy, hydroxyalcoyle, mercapto, formyle, acyle, acylamino, aroylamino, alcoxycarbonylamino, amino, alkylamino, dialkylamino, carboxy, alcoxycarbonyle, carbamoyle, dialcoylcarbamoyle, cyano et trifluorométhyle, étant entendu que les radicaux alcoyles et les portions alcoyles des autres radicaux contiennent 1 à 4 atomes de carbone, que les radicaux alcényles et alcynyles contiennent 3 à 8 atomes de carbone et les radicaux aryles sont les radicaux phényles ou α- ou β-naphtyles.

D'un intérêt tout particulier sont les produits de formule générale (I) dans laquelle R représente un atome d'hydrogène ou un radical acétyle, R₁ représente un radical benzoyle ou t.butoxycarbonylamino et Ar représente un radical phényle.

Les produits de formule générale (I) dans laquelle R₁ représente un radical benzoyle correspondent au taxol et au désacétyl-10 taxol et les produits de formule générale (I) dans laquelle R₁ représente un radical t.butoxycarbonyle correspondent à ceux qui font l'objet du brevet européen EP 0 253 738.

Selon le procédé qui est décrit dans la demande internationale PCE WO 92/09589, les dérivés de formule générale (I) peuvent être obtenus par :
- condensation d'un dérivé de l'oxazolidine de formule générale: 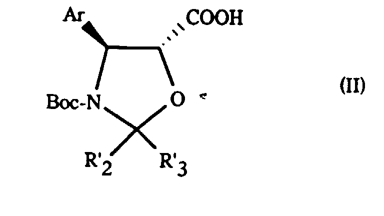 dans laquelle Ar est défini comme précédemment, Boc représente le radical t.butoxycarbonyle et R'₂ et R'₃, identiques ou différents, représentent un radical alcoyle contenant 1 à 4 atomes de carbone éventuellement substitué par un ou plusieurs radicaux aryles, ou un radical aryle, ou bien R'₂ et R'₃ forment ensemble avec l'atome de carbone auquel ils sont liés un cycle ayant de 4 à 7 chaînons, sur la baccatine III ou la désacétyl-10 baccatine III protégée de formule générale : 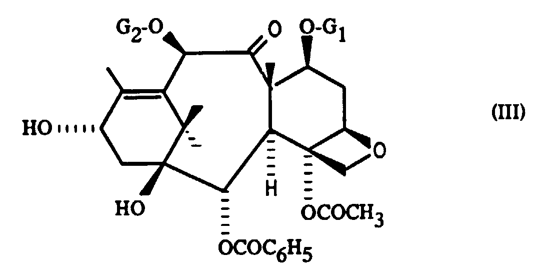 dans laquelle G₁ représente un groupement protecteur de la fonction hydroxy et G₂ représente un radical acétyle ou un groupement protecteur de la fonction hydroxy, pour obtenir un produit de formule générale : 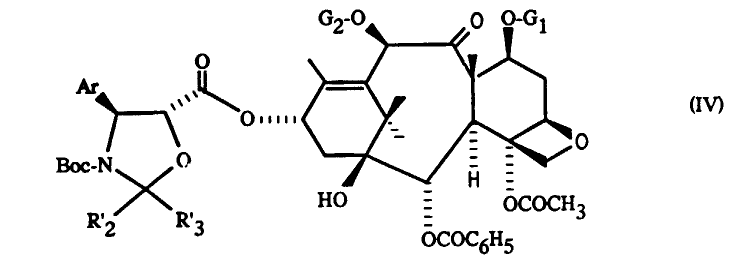 dans laquelle Ar, R'₂, R'₃, G₁, G₂ et Boc sont définis comme précédemment,
- traitement en milieu acide du produit de formule générale (IV) dans des conditions qui sont sans effet sur G₁ et G₂ pour obtenir le produit de formule générale: 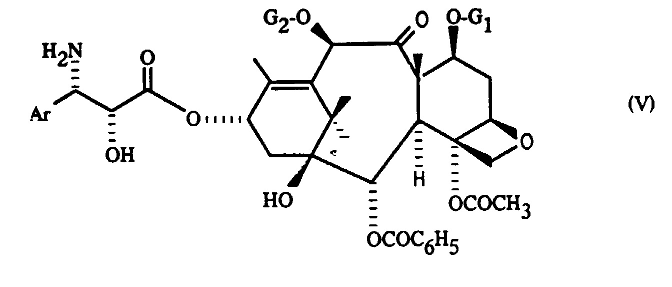 dans laquelle Ar, G₁ et G₂ sont définis comme précédemment,
- traitement du produit de formule générale (V) par un réactif convenable pour introduire un radical benzoyle ou R₂-O-CO-, pour obtenir un produit de formule générale : 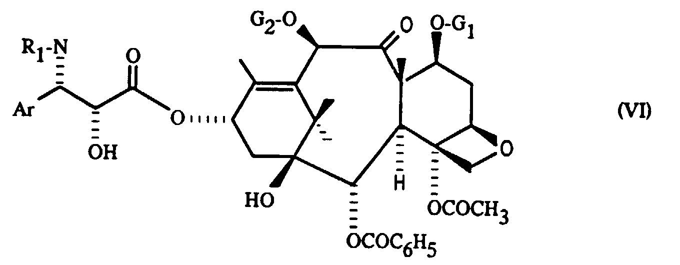 dans laquelle Ar, R₁, G₁ et G₂ sont définis comme précédemment, et
- remplacement des groupements protecteurs G₁ et G₂ du produit de formule générale (VI) par des atomes d'hydrogène pour obtenir le produit de formule générale (I).

Il a maintenant été trouvé, et c'est ce qui fait l'objet de la présente invention, que les produits de formule générale (I) peuvent être obtenus par :
- condensation d'un acide de formule générale : 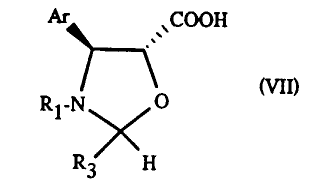 dans laquelle Ar et R₁ sont définis comme précédemment, et R₃ représente un atome d'hydrogène ou un radical alcoxy contenant 1 à 4 atomes de carbone ou un radical aryle éventuellement substitué ou d'un dérivé de cet acide, sur la baccatine III ou la désacétyl-10 baccatine III de formule générale (III) dans laquelle G₁ représente un groupement protecteur de la fonction hydroxy et G₂ représente un radical acétyle ou un groupement protecteur de la fonction hydroxy, pour obtenir un produit de formule générale : 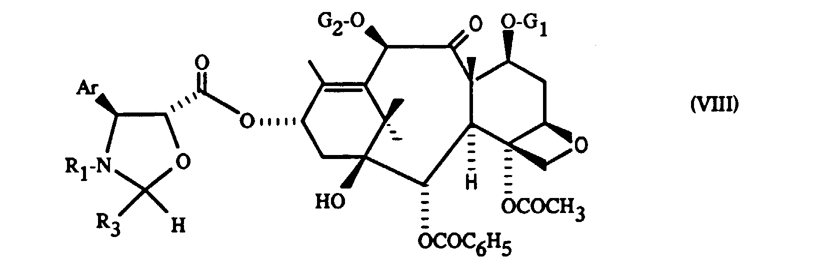 dans laquelle Ar, R₁, R₃, G₁ et G₂ sont définis comme précédemment,
- déprotection de la chaîne latérale et éventuellement des fonctions hydroxy protégées par G₁ et G₂ pour obtenir un produit de formule générale : 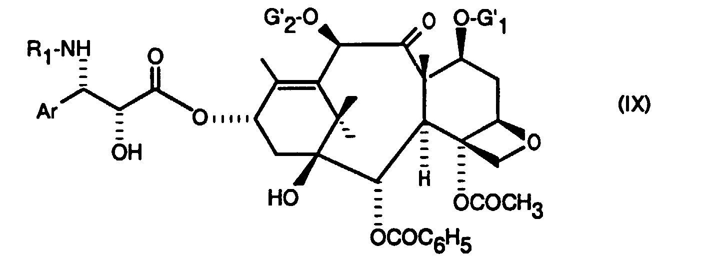 dans laquelle Ar et R₁ sont définis comme précédemment, G'₁ représente un atome d'hydrogène ou un groupement protecteur de la fonction hydroxy et G'₂ représente un atome d'hydrogène ou un radical acétyle ou un groupement protecteur de la fonction hydroxy, puis
- éventuellement remplacement des groupements protecteurs G'₁ et éventuellement G'₂ du produit de formule générale (IX) par des atomes d'hydrogène pour obtenir un produit de formule générale (I).

Selon l'invention, l'estérification du produit de formule générale (III) est effectuée au moyen d'un acide de formule générale (VII) éventuellement sous forme d'anhydride ou sous forme d'halogénure ou d'anhydride mixte.

De préférence, on utilise un acide de formule générale (VII), ou ses dérivés activés, dans laquelle R₃ représente un atome d'hydrogène ou un radical alcoxy contenant 1 à 4 atomes de carbone ou un radical phényle éventuellement substitué par un ou plusieurs radicaux électro-donneurs choisis plus particulièrement dans le groupe des radicaux alcoxy contenant 1 à 4 atomes de carbone.

L'estérification au moyen d'un acide de formule générale (VII) peut être effectuée en présence d'un agent de condensation tel qu'un carbodiimide comme le dicyclohexylcarbodiimide ou un carbonate réactif comme le dipyridyl-2 carbonate et d'un agent d'activation tel qu'une aminopyridine comme la diméthylamino-4 pyridine ou la pyrrolidino-4 pyridine en opérant dans un solvant organique choisi parmi les éthers tels que le tétrahydrofuranne, l'éther diisopropylique, le méthyl t.butyléther ou le dioxanne, les cétones telles que la méthylisobutylcétone, les esters tels que l'acétate d'éthyle, l'acétate d'isopropyle ou l'acétate de n.butyle, les nitriles tels que l'acétonitrile, les hydrocarbures aliphatiques tels que le pentane, l'hexane ou l'heptane, les hydrocarbures aliphatiques halogénés tels que le dichlorométhane ou le dichloro-1,2 éthane ou les hydrocarbures aromatiques tels que le benzène, le toluène, les xylènes, l'éthylbenzène, l'isopropylbenzène ou le chlorobenzène à une température comprise entre -10 et 90°C. Il est particulièrement avantageux d'effectuer l'estérification en opérant dans un solvant aromatique à une température voisine de 20°C.

L'estérification peut aussi être réalisée en utilisant l'acide de formule générale (VII) sous forme d'anhydride de formule : 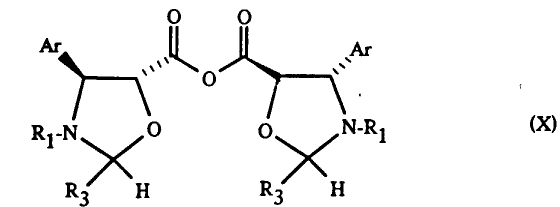 dans laquelle Ar, R₁ et R₃ sont définis comme précédemment, en présence d'un agent d'activation tel qu'une aminopyridine comme la diméthylamino-4 pyridine ou la pyrrolidino-4 pyridine en opérant dans un solvant organique choisi parmi les éthers tels que le tétrahydrofuranne, l'éther diisopropylique, le méthyl t.butyléther ou le dioxanne, les cétones telles que la méthylisobutylcétone, les esters tels que l'acétate d'éthyle, l'acétate d'isopropyle ou l'acétate de n.butyle, les nitriles tels que l'acétonitrile, les hydrocarbures aliphatiques tels que le pentane, l'hexane ou l'heptane, les hydrocarbures aliphatiques halogénés tels que le dichlorométhane ou le dichloro-1,2 éthane ou les hydrocarbures aromatiques tels que le benzène, le toluène, les xylènes, l'éthylbenzène, l'isopropylbenzène ou le chlorobenzène à une température comprise entre 0 et 90°C.

L'estérification peut aussi être réalisée en utilisant l'acide de formule générale (VII) sous forme d'halogénure ou sous forme d'anhydride mixte de formule générale : 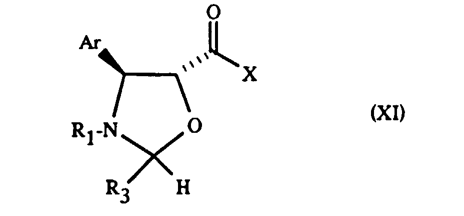 dans laquelle Ar, R₁ et R₃ sont définis comme précédemment et X représente un atome d'halogène ou un radical acyloxy ou aroyloxy, éventuellement préparé in situ, en présence d'une base qui est de préférence une base organique azotée telle qu'une amine aliphatique tertiaire comme la triéthylamine, la pyridine, une aminopyridine comme la diméthylamino-4 pyridine ou la pyrrolidino-4 pyridine en opérant dans un solvant organique inerte choisi parmi les éthers tels que le tétrahydrofuranne, l'éther diisopropylique, le méthyl t.butyléther ou le dioxanne, les cétones, les esters tels que l'acétate d'éthyle, l'acétate d'isopropyle ou l'acétate de n.butyle, les nitriles tels que l'acétonitrile, les hydrocarbures aliphatiques tels que le pentane, l'hexane ou l'heptane, les hydrocarbures aliphatiques halogénés tels que le dichlorométhane ou le dichloro-1,2 éthane et les hydrocarbures aromatiques tels que le benzène, le toluène, les xylènes, l'éthylbenzene, l'isopropylbenzène ou le chlorobenzène à une température comprise entre 10 et 80°C, de préférence voisine de 20°C.

De préférence, on utilise un dérivé activé de formule générale (XI) dans laquelle X représente un atome d'halogène ou un radical acyloxy contenant 1 à 5 atomes de carbone ou aroyloxy dans lequel la partie aryle est un radical phényle éventuellement substitué par 1 à 5 atomes ou radicaux, identiques ou différents, choisis parmi les atomes d'halogène (chlore, brome) et les radicaux nitro, méthyle ou méthoxy.

La déprotection de la chaîne latérale peut être effectuée en présence d'un acide minéral (acide chlorhydrique, acide sulfurique) ou organique (acide acétique, acide méthanesulfonique, acide trifluorométhanesulfonique, p.toluènesulfonique) utilisé seul ou en mélanges, en opérant dans un solvant organique choisi parmi les alcools (méthanols éthanol, propanol, isopropanol), les éthers (tétrahydrofuranne, éther diisopropylique, méthyl t.butyléther), les esters (acétate d'éthyle, acétate d'isopropyle, acétate de n.butyle), les hydrocarbures aliphatiques (pentane, hexane, heptane), les hydrocarbures aliphatiques halogénés (dichlorométhane, dichloro-1,2 éthane), les hydrocarbures aromatiques (benzène, toluène, xylènes) et les nitriles (acétonitrile) à une température comprise entre -10 et 60°C, de préférence entre 15 et 30°C. L'acide minéral ou organique peut être utilisé en quantité catalytique ou stoechiométrique ou en excès.

La déprotection peut être également réalisée dans des conditions oxydantes en Utilisant par exemple le nitrate d'ammonium et de cérium IV dans un mélange acétonitrile-eau ou la dichloro-2,3 dicyano-5,6 benzoquinone-1,4 dans l'eau. La déprotection peut être également réalisée dans des conditions réductrices, par exemple par hydrogénolyse en présence d'un catalyseur.

Les groupements protecteurs G₁ et G₂ sont de préférence des radicaux trichloro-2,2,2 éthoxycarbonyle, (trichlorométhyl-2 propoxy)-2 carbonyle ou des radicaux trialkylsilyle, dialkylarylsilyle, alkyldiarylsilyle ou triarylsilyle dans lesquels les parties alkyles contiennent 1 à 4 atomes de carbone et les parties aryles sont de préférence des radicaux phényles.

Le remplacement des groupements protecteurs G₁ et éventuellement G₂ représentant un radical silylé par des atomes d'hydrogène peut être effectué simultanément avec la déprotection de la chaîne latérale.

Le remplacement des groupements protecteurs G₁ et éventuellement G₂ représentant un radical trichloro-2,2,2 éthoxycarbonyle ou (trichlorométhyl-2 propoxy)-2 carbonyle, est effectué par le zinc, éventuellement associé à du cuivre, en présence d'acide acétique à une température comprise entre 20 et 60°C ou au moyen d'un acide minéral ou organique tel que l'acide chlorhydrique ou l'acide acétique en solution dans un alcool aliphatique contenant 1 à 3 atomes de carbone ou dans un ester aliphatique tel que l'acétate d'éthyle, l'acétate d'isopropyle ou l'acétate de n.butyle en présence de zinc éventuellement associé à du cuivre.

Ce remplacement peut aussi être effectué par réduction électrolytique.

L'acide de formule générale (VII) peut être obtenu par saponification en milieu basique d'un ester de formule générale : 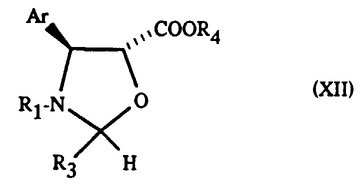 dans laquelle Ar, R₁ et R₃ sont définis comme précédemment et R₄ représente un radical alcoyle contenant 1 à 4 atomes de carbone éventuellement substitué par un radical phényle.

Généralement la saponification est effectuée au moyen d'une base minérale telle qu'un hydroxyde de métal alcalin (lithium, potassium, sodium), un carbonate ou bicarbonate de métal alcalin (bicarbonate de sodium, carbonate ou bicarbonate de potassium) en milieu hydro-alcoolique tel qu'un mélange méthanol-eau à une température comprise entre 10 et 40°C, de préférence voisine de 20°C.

L'ester de formule générale (XII) peut être obtenu par action d'un aldéhyde de formule générale:

R₃-CHO (XIII)

dans laquelle R₃ est défini comme précédemment, éventuellement sous forme d'un dialkylacétal ou d'un alkyléther d'énol ou d'un orthoformiate de formule générale :

HC(OR₃)₃ (XIV)

dans laquelle R₃ est défini comme précédemment, sur un dérivé de la phénylisosérine de formule générale: 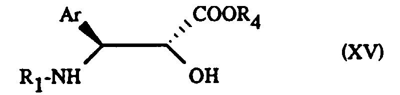 dans laquelle Ar, R₁ et R₄ sont définis comme précédemment, de préférence sous forme 2R,3S en opérant dans un solvant organique inerte en présence d'un acide fort minéral, tel que l'acide sulfurique, ou organique, tel que l'acide p.toluènesulfonique éventuellement sous forme de sel de pyridinium à une température comprise entre 0°C et la température d'ébullition du mélange réactionnel. Les solvants qui conviennent particulièrement bien sont les hydrocarbures aromatiques.

Le dérivé de la phénylisosérine de formule générale (XV) peut être obtenu par acylation d'un dérivé de la phénylisosérine de formule générale : 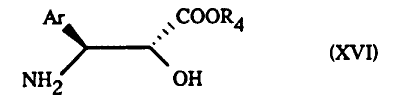 dans laquelle Ar et R₄ sont définis comme précédemment.

L'acylation est effectuée par action du chlorure de benzoyle ou d'un dérivé réactif de formule générale :

R₂-O-CO-Y (XVII)

dans laquelle R₂ est défini comme précédemment et Y représente un atome d'halogène (fluor, chlore) ou un reste -O-R₂ ou -O-CO-O-R₂ en opérant dans un solvant organique tel qu'un ester aliphatique comme l'acétate d'éthyle ou un hydrocarbure aliphatique halogéné comme le dichlorométhane en présence d'une base minérale ou organique telle que le bicarbonate de sodium. Généralement la réaction est effectuée à une température comprise entre 0 et 50°C, de préférence voisine de 20°C.

Le produit de formule générale (XVI) peut être préparé dans les conditions décrites dans la demande internationale PCT W0 92/09589.

L'anhydride de formule générale (X) peut être obtenu en faisant réagir un agent de déshydratation tel que le dicyclohexylcarbodiimide sur l'acide de formule générale (VII) en opérant dans un solvant organique choisi parmi les éthers tels que le tétrahydrofuranne, l'éther diisopropylique, le méthyl t.butyléther ou le dioxanne, les cétones telles que la méthylisobutylcétone, les esters tels que l'acétate d'éthyle, l'acétate d'isopropyle ou l'acétate de n.butyle, les nitriles tels que l'acétonitrile, les hydrocarbures aliphatiques tels que le pentane, l'hexane ou l'heptane, les hydrocarbures aliphatiques halogénés tels que le dichlorométhane ou le dichloro-1,2 éthane ou les hydrocarbures aromatiques tels que le benzène, le toluène, les xylènes, l'éthylbenzène, l'isopropylbenzène ou le chlorobenzène à une température comprise entre 0 et 30°C.

L'acide activé de formule générale (XI) peut être obtenu par action d'un halogénure de sulfuryle, de préférence, le chlorure ou d'un produit de formule générale :

R₅-CO-Z (XVIII)

dans laquelle R₅ représente un radical alcoyle contenant 1 à 4 atomes de carbone ou un radical phényle éventuellement substitué par 1 à 5 atomes ou radicaux, identiques ou différents, choisis parmi les atomes d'halogène et les radicaux nitro, méthyle et méthoxy et Z représente un atome d'halogène, de préférence un atome de chlore sur un acide de formule générale (VII) en opérant dans un solvant organique convenable tel que le tétrahydrofuranne en présence d'une base organique telle qu'une amine tertiaire comme la triéthylamine à une température comprise entre 0 et 30°C.

Le procédé selon la présente invention est particulièrement utile pour préparer les produits de formule générale (I) dans laquelle R représente un atome d'hydrogène ou un radical acétyle, R₁ représente un radical benzoyle ou t.butoxycarbonyle et Ar représente un radical phényle éventuellement substitué.

Les exemples suivants illustrent la présente invention.

### EXEMPLE 1

Une solution de 10,0 g de t.butoxycarbonylamino-3 hydroxy-2 phényl-3 propionate-(2R,3S) de méthyle et de 0,25 g de p.toluènesulfonate de pyridinium dans 200 cm3 de toluène est déshydratée par distillation de 20 cm3 de solvant. On ajoute 6,34 cm3 de diméthylacétal du p.méthoxybenzaldéhyde en 5 minutes sur le mélange réactionnel chauffé à l'ébullition. Pendant l'addition, on distille 50 cm3 de solvant puis on distille encore 100 cm3 de solvant. Après refroidissement à une température voisine de 20°C, on ajoute, en 10 minutes, 80 cm3 de cyclohexane. Le mélange est refroidi à 0-5°C. La bouillie obtenue est filtrée sur verre fritté et le gâteau de filtration est lavé avec 40 cm3 de cyclohexane puis séché sous pression réduite à une température voisine de 20°C. On obtient ainsi, avec un rendement de 74 %, 10,39 g de t.butoxycarbonyl-3 (méthoxy-4 phényl)-2 phényle méthoxycarbonyl-5 oxazolidine-1,3-(2R,4S,5R) dont les caractéristiques sont les suivantes :
- spectre infra-rouge (en comprimé avec KBr) : bandes d'absorption caractéristiques à 3100-3000, 2980, 2960, 2930, 2910, 2840, 1740, 1700, 1614, 1514, 1460, 1435, 1390, 1370, 1245, 1175, 1165, 816, 760 et 700 cm⁻¹
- de résonance magnétique nucléaire du proton (400 MHz ; CDCl₃ ; température: 323°K ; déplacements chimiques δ en ppm ; constantes de couplage J en Hz) : 1,11 (s, 9H) ; 3,60 (s, 3H) ; 3,82 (s, 3H) ; 4,58 (d, J = 5, 1H); 5,42 (d large, J = 5, 1H); 6,38 (s large, 1H) ; 6,92 (d, J = 7,5, 2H) ; 7,30 à 7,45 (mt, 7H).

A une solution de 3,0 g du produit obtenu précédemment dans 27 cm3 de méthanol, on ajoute 14 cm3 d'une solution aqueuse contenant 0,31 g d'hydroxyde de lithium monohydraté. On agite pendant 2 heures à une température voisine de 20°C. Le méthanol est éliminé par distillation sous pression réduite puis on ajoute 40 cm3 de dichlorométhane. Sous forte agitation, le mélange réactionnel est acidifié par addition d'acide chlorhydrique 1N jusqu'à pH = 1. Après décantation, la phase aqueuse est extraite 2 fois par 40 cm3 de dichlorométhane. Les phases organiques réunies sont séchées sur sulfate de sodium. Après filtration et évaporation du solvant, on obtient, avec un rendement de 94,5 %, 2,88 g d'acide t.butoxycarbonyl-3 (méthoxy-4 phényl)-2 phényl-4 oxazolidine-1,3 carboxylique-5-(2R,4S,5R) dont les caractéristiques sont les suivantes:
- spectre infra-rouge (en comprimé avec KBr) : bandes d'absorption caractéristiques à 3325-2675, 2980, 2955, 2935, 2845, 1755, 1700, 1615, 1590, 1515, 1460, 1250, 1175, 1030, 835, 765 et 705 cm⁻¹
- spectre de résonance magnétique nucléaire du proton (250 MHz ; CDCl₃ ; déplacements chimiques δ en ppm ; constantes de couplage J en Hz) : 1,08 (s, 9H) ; 3,82 (s, 3H) ; 4,61 (d, J = 5, 1H); 5,42 (d large, J = 5, 1H); 6,38 (s large, 1H); 6,92 (d, J = 7,5, 2H) ; 7,30 à 7,45 (mt, 7H).

### EXEMPLE 2

A une solution agitée de 1,0 g d'acide t.butoxycarbonyl-3 (méthoxy-4 phényl)-2 phényl-4 oxazolidine-1,3 carboxylique-5-(2R,4S,5R), de 1,34 g d'acétoxy-4 benzoyloxy-2α époxy-5β,20 dihydroxy-1,13α oxo-9 bis-(trichloro-2,2,2 éthoxy) carbonyloxy-7β,10β taxène-11 et de 0,061 g de diméthylamino-4 pyridine dans 7,6 cm3 de toluène anhydre, on ajoute, à 0°C, 0,52 g de dicyclohexylcarbodiimide. On agite pendant 2 heures à une température de 20°C. La dicyclohexylurée est séparée par filtration et lavée avec du toluène. Les phases organiques réunies sont lavées par une solution d'acide chlorhydrique 0,1N, une solution saturée d'hydrogénocarbonate de sodium et séchées sur sulfate de sodium. Après filtration et concentration à sec sous pression réduite, on obtient 2,09 g de t.butoxycarbonyl-3 (méthoxy-4 phényl)-2 phényl-4 oxazolidine-1,3 carboxylate-5-(2R,45,5R) d'acétoxy-4 benzoyloxy-2α époxy-5β,20 hydroxy-1 oxo-9 bis-(trichloro-2,2,2 éthoxy) carbonyloxy-7β,10β taxène-11 yle-13α brut dont les caractéristiques sont les suivantes :
- spectre infra-rouge (CHCl₃) : bandes d'absorption caractéristiques à 3575, 1765, 1740, 1725, 1710, 1615, 1515, 1455, 1250, 1175, 980, 710 et 700 cm⁻¹
- de résonance magnétique nucléaire du proton (400 MHz ; CDCl₃ ; température: 323°K ; déplacements chimiques δ en ppm ; constantes de couplage J en Hz) : 1,09 (s, 9H) ; 1,18 (s, 3H) ; 1,27 (s, 3H) ; 1,67 (s, 3H) ; 1,72 (s, 1H) ; 1,82 (s, 3H) ; 1,90 (s, 3H) ; 2,02 (m, 1H) ; 2,13 (dd, J = 15 et 9, 1H) ; 2,25 (dd, J = 15 et 9, 1H) ; 2,60 (mt, 1H); 3,83 (d, J = 7, 1H) ; 3,83 (s, 3H) ; 4,12 (d, J = 8, 1H); 4,26 (d, J = 8, 1H) ; 4,60 (d, J = 5, 1H); 4,61 (d, J = 12, 1H); 4,78 (ab limite, J = 11, 2H) ; 4,90 (d large, J = 10, 1H); 4,90 (d, J = 12, 1H); 5,45 (d large, J = 5, 1H); 5,50 (dd, J = 11 et 7, 1H); 5,66 (d, J = 7, 1H); 6,12 (t, J = 9, 1H); 6,18 (s, 1H); 6,39 (s large) ; 6,94 (d, J = 7,5, 2H) ; 7,42 (d, J = 7,5, 2H) ; 7,35 à 7,50 (mt, 5H) ; 7,49 (t, J = 5, 2H) ; 7,63 (t, J = 7,5, 1H); 8,03 (d, J =77,5, 2H).

A une solution de 0,161 g du produit obtenu précédemment dans 2,1 cm3 d'acétate d'éthyle on ajoute 9 µl d'une solution aqueuse d'acide chlorhydrique à 37 % (p/p). On agite pendant 3 heures à une température voisine de 20°C. Un dosage par chromatographie liquide à haute performance montre que le rendement en t.butoxycarbonylamino-3 phényl-3 hydroxy-2 propionate-(2R,3S) d'acétoxy-4 benzoyloxy-2α époxy-5β,20 hydroxy-1 oxo-9 bis-(trichloro-2,2,2 éthoxy) carbonyloxy-7β,10β taxène-11 yle-13α est de 95 %.

Le t.butoxycarbonylamino-3 phényl-3 hydroxy-2 propionate-(2R,3S) d'acétoxy-4 bemoyloxy-2α époxy-5β,20 hydroxy-1 oxo-9 bis-(trichloro-2,2,2 éthoxy) carbonyloxy-7β,10β taxène-11 yle-13α est transformé en t.butoxycarbonylamino-3 phényl-3 hydroxy-2 propionate-(2R,3S) d'acétoxy-4 benzoyloxy-2α époxy-5β,20 oxo-9 trihydroxy-1,7β,10β taxène-11 yle-13α (ou Taxotère) dans les conditions décrites dans le brevet EP 0 253 738.

### EXEMPLE 3

Une solution de 2,43 g de t.butoxycarbonylamino-3 hydroxy-2 phényl-3 propionate-(2R,3S) de méthyle et de 0,059 g de p.toluènesulfonate de pyridinium dans 60 cm3 de toluène est déshydratée en distillant 5 cm3 de solvant. On ajoute, en 15 minutes, une solution de 1,7 g de diméthylacétal du diméthoxy-3,4 benzaldéhyde dans 14 cm3 de toluène sur le mélange réactionnel chauffé à l'ébullition. Pendant l'addition, on disitlle 15 cm3 de toluène puis on distille encore 25 cm3. Après refroidissement à une température voisine de 20°C, on ajoute, sous agitation, 40 cm3 d'eau Après décantation, la phase organique est séchée sur sulfate de magnésium. Après filtration et concentration à sec, le résidu est repris par 8 cm3 de diisopropyléther. Le produit qui cristallise est séparé par filtration, rincé avec du diipropyléther puis séché sous pression réduite. On obtient ainsi, avec un rendement de 50 %, 1,7 g de t.butoxycarbonylamino-3 (diméthoxy-3,4 phényl)-2 phényl-4 méthoxycarbonyl-5 oxazolidine-1,3-(2R,4S,5R) dont les caractéristiques sont les suivantes :
- spectre infra-rouge (comprimés en mélange avec KBr) : bandes d'absorption caractéristiques à 3085, 3065, 3030, 2975, 2935, 2840, 1740, 1700, 1600, 1520, 1495, 1455, 1425, 1265, 1175, 1025, 800, 755 et 700 cm⁻¹
- spectre de résonance magnétique nucléaire du proton (300 MHz ; DMSO d₆ ; déplacements chimiques δ en ppm ; constantes de couplage J en Hz) ; 1,00 (s, 9H) ; 3,58 (s, 3H) ; 3,80 (s, 3H) ; 3,83 (s, 3H) ; 4,68 (d, J = 4, 1H) ; 5,31 (mf, 1H) ; 6,34 (mf, 1H); 6,95 à 7,10 (mt, 3H) ; 7,35 à 7,50 (mt, 5H).

A une solution de 1,63 g de l'ester ainsi obtenu dans 25 cm3 de méthanol et 7 cm3 d'eau distillée, on ajoute 0,24 g de potasse à 86 %. On agite pendant 40 minutes à une température voisine de 20°C. Après élimination du méthanol par distillation sous pression réduite et acidification du milieu à pH = 3-4 par addition d'acide chlorhydrique 1N, le précipité obtenu est séparé par filtration. Le gâteau de filtration est lavé à l'eau puis séché. On obtient ainsi, avec un rendement de 92 %, 1,45 g d'acide t.butoxycarbonyl-3 (diméthoxy-3,4 phényl)-2 phényl-4 oxazolidine-1,3 carboxylique-5-(2R,4S,5R) dont la pureté est de 95 %, et dont les caractéristiques sont les suivantes :
- spectre infra-rouge (comprimés en mélange avec KBr) : bandes d'absorption caractéristiques à 3225, 3030, 3005, 2975, 2930, 2840, 1740, 1710, 1610, 1600, 1515, 1465, 1455, 1260, 1175, 1020, 760 et 700 cm⁻¹
- de résonance magnétique nucléaire du proton (250 MHz ; DMSO d₆ ; déplacements chimiques δ en ppm ; constantes de couplage J en Hz) : 1,00 (s, 9H) ; 3,78 (s, 3H) ; 3,81 (s, 3H) ; 4,55 (d, J = 4, 1H); 5,23 (mf, 1H); 6,29 (mf, 1H); 6,90 à 7,10 (mt,3H) ; 7,30 à 7,50 (mt, 5H).

### EXEMPLE 4

A une suspension agitée de 0,155 g d'acide t.butoxycarbonyl-3 (diméthoxy-3,4 phényl)-2 phényl-4 oxazolidine-1,3 carboxylique-5-(2R,4S,5R) et de 0,24 g d'acétoxy-4 benzoyloxy-2α époxy-5β,20 dihydroxy-1,13α oxo-9 bis-(trichloro-2,2,2 éthoxy) carbonyloxy-7β,10β taxéne-11 dans 2,5 cm3 de toluène anhydre, on ajoute, en une seule fois, à 0°C, 0,076 g de dicyclohexylcarbodiimide et 0,0075 g de diméthylamino-4 pyridine. On agite pendant 1 heure à 0°C. La dicyclohexylurée formée est séparée par filtration. Le gâteau est lavé avec du toluène. Les phases toluéniques réunies sont lavées successivement avec une solution aqueuse saturée de bicarbonate de sodium puis avec de l'eau. Après séchage et concentration à sec sous pression réduite, on obtient, avec un rendement quantitatif, 0,435 g de t.butoxycarbonyl-3 (diméthoxy-3,4 phényl)-2 phényl-4 oxazolidine-1,3 carboxylate-5-(2R,4S,5R) d'acétoxy-4 benzoyloxy-2α époxy-5β,20 hydroxy-1 oxo-9 bis-(trichloro-2,2,2 éthoxy) carbonyloxy-7β,10β taxène-11 yle-13α dont les caractéristiques sont les suivantes :
- spectre infra-rouge (CCl₄) : bandes d'absorption caractéristiques à 3580, 3550-3375, 3090, 3070, 3030, 1765, 1740, 1730, 1715, 1605, 1520, 1500, 1465, 1455, 1265, 1250, 1180, 1035, 985, 710 et 695 cm⁻¹
- de résonance magnétique nucléaire du proton (400 MHz ; CDCl₃ ; température : 323°K ; déplacements chimiques δ en ppm ; constantes de couplage J en Hz) : 1,10 (s, 9H) ; 1,17 (s, 3H) ; 1,25 (s, 3H) ; 1,66 (s, 3H) ; 1,70 (s, 1H); 1,82 (s, 3H) ; 1,90 (s, 3H) ; 2,02 (mt, 1H) ; 2,13 (dd, J = 15 et 9, 1H) ; 2,24 (dd, J = 15 et 9, 1H) ; 2,60 (mt, 1H); 3,83 (d, J = 7, 1H); 3,89 (s, 3H) ; 3,93 (s, 3H) ; 4,12 (d, J = 8, 1H) ; 4,26 (d, J = 8, 1H) ; 4,60 (d, J = 4,5, 1H) ; 4,60 (d, J = 12, 1H) ; 4,78 (ab limite, 2H) ; 4,89 (d large, J = 10, 1H) ; 4,90 (d, J = 12, 1H) ; 5,46 (d large, J = 4,5, 1H) ; 5,50 (dd, J = 11 et 7, 1H); 5,66 (d, J = 7, 1H); 6,13 (t, J = 9, 1H); 6,15 (s, 1H) ; 6,39 (s, 1H) ; 6,90 (d, J = 7,5, 1H) ; 7,03 (d, J = 1, 1H) ; 7,07 (dd, J = 7,5 et 1, 1H); 7,35 à 7,50 (mt, 5H) ; 7,48 (t, J = 7,5, 2H) ; 7,62 (t, J = 7,5, 1H); 8,03 (d, J = 7,5, 2H).

A une solution de 0,223 g de l'ester obtenu ci-dessus dans 2,5 cm3 de méthanol, on ajoute 2 µl d'acide méthanesulfonique. On agite pendant 2 heures 30 minutes à une température voisine de 20°C. Le dosage par chromatographie liquide à haute performance montre que le rendement en t.butoxycarbonylamino-3 phényl-3 hydroxy-2 propionate-(2R,3S) d'acétoxy-4 benzoyloxy-2α époxy-5β,20 hydroxy-1 oxo-9 bis-(trichloro-2,2,2 éthoxy) carbonyloxy-7β,10β taxène-11 yle-13α est de 88%.

### EXEMPLE 5

Une solution de 0,497 g de t.butoxycarbonylamino-3 hydroxy-2 phényl-3 propionate-(2R,3S) de méthyle, de 0,021 g de p.toluènesulfonate de pyridinium et de 0,295 g de diméthoxy-2,4 benzaldéhyde dans 20 cm3 de toluène anhydre est chauffée au reflux pendant 24 heures. L'eau formée pendant la réaction est éliminée au moyen d'un Dean-Stark. Après refroidissement à une température voisine de 20°C, la solution est lavée avec une solution aqueuse d'hydrogénosulfite de sodium à 37 % (p/p) puis avec une solution aqueuse saturée de bicarbonate de sodium. Après concentration de la phase organique sous pression réduite, on obtient, avec un rendement de 80 %, 0,700 g de t.butoxycarbonyl-3 (diméthoxy-2,4 phényl)-2 phényl-4 méthoxycarbonyl-5 oxazolidine-1,3-(4S,5R) sous forme d'un mélange des formes diastéréoisomériques A et B quasi équimoléculaire dont les caractéristiques sont les suivantes :
- spectre infra-rouge (CCl₄) : bandes d'absorption caractéristiques à 3095, 3070, 3035, 2980, 2955, 2935, 2840, 1760, 1745, 1710, 1615, 1590, 1510, 1465, 1455, 1435, 1210, 1160, 1040, 835 et 700 cm⁻¹
- spectre de résonance magnétique nucléaire du proton (250 MHz ; DMSO d₆ ; déplacements chimiques δ en ppm ; constantes de couplage J en Hz) : 1,00 (s, -C(CH₃)₃ de B) ; 1,22 (s, -C(CH₃)₃ de A) ; 3,55 (mf, -COOCH₃ ou -OCH₃ de B) ; 3,87 à 3,85 (mt, -COOCH₃ ou -OCH₃ de A et B) ; 4,64 (d, J = 4,5, -H5 de B) ; 5,01 (d, J =2,5, -H5 de A) ; 5,21 (d, J = 2,5, -H4 de A) ; 5,26 (d, J = 4,5, -H4 de B) ; 6,46 [dd, J = 7,5 et 1,5, -C₆H₅ en 2 (-H5) de A] ; 6,52 (s, -H2 de A) ; 6,50-6,65 [mt, -H2 et -C₆H₅ en 2 (-H5 et -H3) de B + -C₆H₅ en 2 (-H3) de A] ; 7,00 [d, J = 7,5, -C₆H₅ en 2 (-H6) de B] ; 7,30 à 7,55 [mt, 5H, -C₆H₅ en 4 (H2 à -H6) de A et B].

A une solution de 0,700 g de l'ester obtenu précédemment dans un mélange de 9 cm3 de méthanol et de 3 cm3 d'eau distillée, on ajoute 0,073 g d'hydroxyde de lithium monohydraté. On agite pendant 3 heures 30 minutes à une température voisine de 20°C. Le méthanol est éliminé par distillation sous pression réduite. La phase aqueuse est lavée avec du toluène puis est acidifiée jusqu'à pH = 3-4 par addition d'une solution aqueuse d'acide chlorhydrique 1N. Le précipité obtenu est séparé par filtration et le gâteau de filtration est lavé abondamment à l'eau jusqu'à neutralité puis séché sous pression réduite. On obtient ainsi, avec un rendement de 74 %, 0,450 g d'acide t.butoxycarbonyl-3 (diméthyl-2,4 phényl)-2 phényl-4 oxazolidine-1,3 carboxylique-5-(4S,5R) sous forme d'un mélange des formes diastéréoisomériques A et B quasi équimoléculaire dont les caractéristiques sont les suivantes :
- spectre infra-rouge (en comprimé avec KBr) : bandes d'absorption caractéristiques à 3300-2700, 2700-2250, 3070, 3030, 3005, 2975, 2940, 2840, 1710, 1615, 1590, 1510, 1460, 1210, 1160, 1035, 835 et 700 cm⁻¹
- spectre de résonance magnétique nucléaire du proton (200 MHz ; DMSO d₆ ; température : 393°K ; déplacements chimiques δ en ppm ; constantes de couplage J en Hz ; mélange des 2 diastéréoisomères dans la proportion 55/45) : 1,00 (s, -C(CH₃)₃ de B) ; 1,25 (s, -C(CH₃)₃ de A) ; 3,75 à 3,85 (mt, 6H, -OCH₃ de A et B) ; 4,43 (d, J = 5, -H5 de B) ; 4,77 (d, J = 2, -H5 de A) ; 5,21 (d, J = 2, -H4 de A) ; 5,21 (d, J = 2, -H4 de B) ; 6,42 [dd, J = 7,5 et 1,5, -C₆H₅ en 2 (-H5) de A] ; 6,49 (s, -H2 de A) ; 6,45-6,60 [mt, -H2 et -C₆H₅ en 2 (-H5 et -H3) de B + -C₆H₅ en 2 (-H3) de A] ; 7,02 [d, J = 7,5, -C₆H₅ en 2 (-H6) de A] ; 7,15 [d, J = 7,5, -C₆H₅ en 2 (H6) de B] ; 7,25 à 7,50 [mt, 5H, -C₆H₅ en 4 (-H2 à -H6) de A et B].

### EXEMPLE 6

A une suspension agitée de 1,671 g d'acide t.butoxycarbonyl-3 (diméthoxy-2,4 phényl)-2 phényl-4 oxazolidine-1,3 carboxylique-5-(4S,5R) et de 1,003 g d'acétoxy-4 benzoyloxy-2α époxy-5β,20 dihydroxy-1,13α oxo-9 bis-(trichloro-2,2,2 éthoxy) carbonyloxy-7β,10β taxène-11 dans 8 cm3 de toluène anhydre on ajoute, en une seule fois, à 0°C, 0,656 g de dicyclohexylcarbodiimide et 0,0287 g de diméthylamino-4 pyridine. On agite pendant 10 minutes à 0°C puis pendant 5 heures à une température voisine de 20°C. La dicyclohexylurée formée est séparée par filtration et lavée avec du toluène. Les phases toluéniques réunies sont lavées avec une solution aqueuse saturée de bicarbonate de sodium, puis à l'eau. Après séchage, filtration et concentration à sec sous pression réduite, on obtient 1,623 g de t.butoxycarbonyl-3 (diméthoxy-2,4 phényl)-2 phényl-4 oxazolidine-1,3 carboxylate-5-(4S,5R) d'acétoxy-4 benzoyloxy-2α époxy-5β,20 hydroxy-1 oxo-9 bis-(trichloro-2,2,2 éthoxy) carbonyloxy-7β,10β taxène-11 yle-13α brut sous forme d'un mélange diaétéréoisomérique dont on sépare les constituants par chromatographie liquide sur gel de silice en éluant avec un mélange acétate d'éthyle-cyclohexane (75-25 en volumes).

Un des deux diastéréoisomères présente les caractéristiques suivantes :
- spectre de résonance magnétique nucléaire du proton (400 MHz ; CDCl₃ ; déplacements chimiques δ en ppm ; constantes de couplage J en Hz) : 1,20 (s, 3H) ; 1,25 (s, 9H) ; 1,30 (s, 3H) ; 1,76 (s, 1H); 1,85 (s, 3H) ; 2,00 (s, 3H) ; 2,05 (mt, 1H); 2,17 (s, 3H) ; 2,26 (dd, J = 15 et 9, 1H); 2,34 (dd, J = 15 et 9, 1H); 2,60 (mt, 1H); 3,82 (s, 3H) ; 3,92 (s, 3H) ; 3,95 (d, J = 7, 1H); 4,14 (d, J = 8, 1H); 4,30 (d, J = 8, 1H); 4,62 (d, J =12, 1H); 4,80 (ab limite, 2H) ; 4,90 (mt, 1H) ; 4,92 (mt, 1H) ; 4,92 (d, J = 12, 1H) ; 5,36 (d, J = 2, 1H) ; 5,63 (dd, J = 11 et 7, 1H) ; 5,70 (d, J = 7, 1H); 6,28 (s, 1H); 6,34 (t, J = 9, 1H); 6,43 (dd, J = 7,5 et 1,5, 1H); 6,51 (d, J = 1,5, 1H); 6,69 (s, 1H); 7,16 (d, J = 7,5, 1H); 7,35 à 7,50 (mt, 3H) ; 7,48 (t, J = 7,5, 2H) ; 7,67 (d, J = 7,5, 2H); 7,63 (t, J = 7,5, 1H); 8,04 (d, J = 7,5, 2H).

L'autre diastéréoisomère présente les caractéristiques suivantes :
- spectre infra-rouge (CCl₄) : bandes d'absorption caractéristiques à 3580, 3550-3300, 3070, 3030, 1760, 1740, 1710, 1610, 1590, 1510, 1455, 1435, 1260, 1250, 1210, 1180, 1035, 985, 710 et 700 cm⁻¹
- spectre de résonance magnétique nucléaire du proton (400 MHz ; CDCl₃ ; déplacements chimiques δ en ppm ; constantes de couplage J en Hz) : 1,10 [s, 9H : -C(CH₃)₃] ; 11,16 (s, 3H : -CH₃ 16 ou 17) ; 1,24 (s, 3H : -CH₃ 16 ou 17) ; 1,53 (s, 3H : -CH₃ 19) ; 1,66 (s, 1H : -OH 1) ; 1,82 (s, 3H : -CH₃ 18) ; 2,00 (s, 3H : -COCH₃) ; 2,00 (mt, 1H : -(CH)-H6) ; 2,12 (dd, J = 15 et 9, 1H : -(CH)-H14) ; 2,24 (dd, J = 15 et 9, 1H : -(CH)-H14) ; 2,60 (mt, 1H : -(CH)-H6) ; 3,82 (d, J = 7, 1H : -H3) ; 3,82 (s, 3H : -OCH₃) ; 3,90 (s, 3H : -OCH₃) ; 4,12 (d, J = 8, 1H : -(CH)-H20); 4,26 (d, J = 8, 1H : -(CH)-H20) ; 4,55 (d, J = 4, 1H : -H5') ; 4,62 (d, J = 12, 1H : -O(CH)-H du CCl₃CH₂OCOO en -7) ; 4,78 (ab, J = 11, 2H : O-CH₂ du Cl₃CH₂OCOO en -10) ; 4,89 (d large, J = 10, 1H : -H5) ; 4,89 (d; J = 12, 1H : -O(CH)-H du Cl₃CCH₂OCOO en -7) ; 5,46 (d large, J = 4, 1H : -H4') ; 5,50 (dd, J = 11 et 7, 1H : -H7) ; 5,65 (d, J = 7, 1H : -H2) ; 6,05 (t, J = 9, 1H : -H13) ; 6,16 (s, 1H: -H10) ; 6,50 [mt, 2H : -C₆H₅ en 2' (-H3 et -H5)] ; 6,72 (mf, 1H: -H2') ; 7,22 [d, J = 7,5, 1H : -C₆H₅ en 2' (-H6)] ; 7,30 à 7,50 [mt, 5H : -C₆H₅ en 4' (-H2 à -H6)] ; 7,48 [t, J = 7,5, 2H : -OCOC₆H₅ (-H3 et -H5)] ; 7,63 [t, J = 7,5, 1H : -OCOC₆H₅ (-H4)] ; 8,03 [d, J = 7,5, 2H : -OCOC₆H₅ (-H2 et -H6)].

A une solution de 1,623 g de l'ester brut obtenu ci-dessus dans 20 cm3 de méthanol, on ajoute 80 µl d'acide méthanesulfonique. On agite pendant 4 heures à une température voisine de 20°C. Le dosage par chromatographie liquide à haute performance montre que le rendement en t.butoxycarbonylamino-3 phényl-3 hydroxy-2 propionate-(2R,3S) d'acétoxy-4 benzoyloxy-2α époxy-5β,20 oxo-9 bis-(trichloro-2,2,2 éthoxy) carbonyloxy-7β,10β taxène-11 yle-13α est de 88 %.

### EXEMPLE 7

Une solution de 10,0 g de t.butoxycarbonylamino-3 hydroxy-2 phényl-3 propionate-(2R,3S) de méthyle, de 1,0 g de p.toluènesulfonate de pyridinium et de 5,7 cm3 de diméthylacétal de benzaldéhyde dans 250 cm3 de toluène anhydre est chauffée au reflux. On distille 200 cm3 de solvant en 2 heures. La solution est refroidie à une température voisine de 20°C et est lavée avec 50 cm3 d'eau. Après décantation, séchage et concentration à sec de la phase organique, le résidu obtenu est repris dans 14 cm3 de diisopropyléther. La bouillie obtenue est filtrée, rincée et essorée. On obtient ainsi, avec un rendement de 65 %, 8,4 g de t.butoxycarbonylamino-3 diphényl-2,4 méthoxycarbonyl-5 oxazolidine-1,3-(2R,4S,5R) sous forme d'un seul diastéréoisomère dont les caractéristiques sont les suivantes :
- spectre infra-rouge (comprimé en mélange avec KBr) : bandes d'absorption caractéristiques à 3250, 3095, 3070, 3030, 2975, 1710, 1500, 1460, 1165, 760 et 700 cm⁻¹
- spectre de résonance magnétique nucléaire du proton (300 MHz ; DMSO d₆ ; déplacements chimiques δ en ppm ; constantes de couplage J en Hz) : 0,95 (s, 9H) ; 4,26 (mf, 1H) ; 5,10 (mf, 1H); 6,20 (s, 1H) ; 7,25-7,55 (mt, 5H).

A une solution de 7,07 g de l'ester obtenu précédemment dans 88 cm3 de méthanol et 22 cm3 d'eau, on ajoute 1,26 g de potasse à 86 %. On agite pendant une nuit à une température voisine de 25°C. Le méthanol est éliminé par distillation sous pression réduite. On acidifie par addition d'acide chlorhydrique 1N jusqu'à pH = 2. Le précipité obtenu est séparé par filtration, lavé abondamment à l'eau jusqu'à neutralité puis séché sous pression réduite. On obtient ainsi, avec un rendement quantitatif, 7,0 g d'acide t.butoxycarbonyl-3 diphényl-2,4 oxazolidine-1,3 carboxylique-5-(2R,4S,SR) sous forme d'un seul diastéréoisomère dont les caractéristiques sont les suivantes :
- spectre infra-rouge (comprimé en mélange avec KBr) : principales bandes d'absorption caractéristiques à 3080, 3050, 3030, 3005, 2975, 1760, 1695, 1600, 1585, 1490, 1460, 1435, 1175, 760 et 700 cm⁻¹
- spectre de résonance magnétique nucléaire du proton (200 MHz ; DMSO d₆ ; déplacements chimiques δ en ppm ; constantes de couplage J en Hz) ; 0,98 (s, 9H) ; 3,38 (s, 3H) ; 4,71 (d, J = 4, 1H) ; 5,30 (d large, J = 4, 1H) ; 6,38 (s, 1H); 7,25 à 7,55 (mt, 5H).

### EXEMPLE 8

A une suspension agitée de 1,25 g d'acide t.butoxycarbonyl-3 diphényl-2,4 oxazolidine-1,3 carboxylique-5-(2R,4S,5R) et de 1,08 g d'acétoxy-4 benzoyloxy-2α époxy-5β,20 dihydroxy-1,13α oxo-9 bis-(trichloro-2,2,2 éthoxy) carbonyloxy-7β,10β taxène-11 dans 12 cm3 de toluène anhydre, on ajoute 0,70 g de dicyclohexylcarbodiimide et 0,030 g de diméthylamino-4 pyridine. On agite pendant 24 heures à une température voisine de 20°C. La dicyclohexylurée formée est séparée par filtration et lavée par du toluène. Les phases organiques réunies sont lavées avec une solution aqueuse saturée de bicarbonate de sodium. Après séchage et concentration à sec sous pression réduite, on obtient 2,27 g d'un produit brut qui est purifié par chromatographie liquide sur gel de silice en éluant avec un mélange hexane-acétate d'éthyle (1-1 en volumes). On obtient ainsi, avec un rendement de 75%, 1,05 g de t.butoxycarbonyl-3 diphényl-2,4 oxazolidine-1,3 carboxylate-5-(2R,4S,5R) d'acétoxy4 benzoyloxy-2α époxy-5β,20 hydroxy-1 oxo-9 bis-(trichloro-2,2,2 éthoxy) carbonyloxy-7β,10β taxène-11 yle-13α sous forme d'un seul diastéréoisomère dont les caractéristiques sont les suivantes :
- spectre infra-rouge (en comprimé avec KBr) : principales bandes d'absorption caractéristiques à 3250, 3095, 3070, 3030, 2975, 1710, 1500, 1460,1165, 760 et 700 cm⁻¹ ;
- spectre de résonance magnétique nucléaire du proton (400 MHz ; CDCl₃ ; déplacements chimiques δ en ppm ; constantes de couplage J en Hz ) : 1,05 (s, 9H) ; 1,15 (s, 3H) 1,25 (s, 3H) 1,63 (s, 3H) ; 1,73 (s, 1H); 1,80 (s, 3H) ; 1,87 (mf, 3H) ; 2,01 (mt, 1H); 2,08 (dd, J = 15 et 9, 1H); 2,23 (dd, J = 15 et 9, 1H); 2,58 (mt,1H) ; 3,81 (d, J = 7, 1H) ; 4,10 (d, J = 8, 1H) ; 4,26 (d, J = 8, 1H) ; 4,60 (d, J = 12, 1H) ; 4,61 (d, J = 4, 1H); 4,78 (ab, J = 11, 2H) ; 4,87 (d large,j = 10, 1H); 4,90 (d, J = 12, 1H) ; 5,46 (mt, 1H); 5,50 (dd, J= 11 et 7, 1H) ; 5,63 (d, J = 7, 1H) ; 6,13 (mt, 1H); 6,13 (s, 1H); 6,43 (mf, 1H); 7,35 à 7,50 (mt, 10H) ; 7,48 (t, J = 7,5, 2H) ; 7,62 (t, J = 7,5, 1H) ; 8,03 (d, J = 7,5, 2H).

A une solution de 41 mg de l'ester obtenu précédemment dans 0,4 cm3 de méthanol, on ajoute 2,6 µl d'acide méthanesulfonique. On agite pendant 48 heures à une température voisine de 20°C. Le dosage par chromatographie liquide à haute performance montre que l'on obtient le t.butoxycarbonylamino-3 phényl-3 hydroxy-2 propionate-(2R,3S) d'acétoxy4 benzoyloxy-2α époxy-5β,20 hydroxy-1 oxo-9 bis(trichloro-2,2,2 éthoxy) carbonyloxy-7β,10β taxène-11 yle-13α avec un rendement de 50 %.

### EXEMPLE 9

Une solution de 10,0 g de t.butoxycarbonylamino-3 hydroxy-2 phényl-3 propionate-(2R,3S) de méthyle, de 0,334 g de p.toluènesulfonate de pyridinium et de 3,75 cm3 d'orthoformiate de triméthyle dans 70 cm3 de toluène est chauffée au reflux. On distille 4 cm3 de solvant. Après refroidissement à une température voisine de 20°C et filtration, le filtrat est concentré à sec sous pression réduite. Le résidu est repris par 50 cm3 d'hexane. La bouillie obtenue est filtrée, rincée et essorée. On obtient ainsi, avec un rendement de 40 %, 4,6 g de t.butoxycarbonyl-3 méthoxy-2 phényl-4 méthoxycarbonyl-5 oxazolidine-1,3-(4S,5R), sous forme d'un mélange des diastéréoisomères dont les caractéristiques sont les suivantes :
- spectre infra-rouge (CH₂Cl₂) : bandes d'absorption caractéristiques à 2980, 2955, 2935, 2840, 1760, 1745, 1710, 1495, 1460, 1440, 1175, 1080 et 1065 cm⁻¹
- spectre de résonance magnétique nucléaire du proton (300 MHz ; DMSO d₆ ; température: 393°K ; déplacements chimiques δ en ppm ; constantes de couplage J en Hz) sur le mélange 65/35 des diastéréoisomères : 1,22 (s, 3H) ; 1,32 (s, 3H) ; 3,34 (s, 3H) ; 3,43 (s, 3H) ; 3,75 (s, 3H) ; 4,55 (d, J = 3, 1H); 4,68 (d, J = 8, 1H); 4,98 (d, J = 8, 1H) ; 5,17 (d, J = 3, 1H); 6,10 (s, 1H)n 6,13 (s, 1H) ; 7,20 à 7,50 (mt, 5H).

A une solution de 11,27 g du produit obtenu ci-dessus dans 85 cm3 de méthanol et 28 cm3 d'eau, on ajoute 16,1 g d'hydroxyde de lithium monohydraté. On agite pendant 30 minutes à une température voisine de 20°C. Le méthanol est éliminé par distillation sous pression réduite puis on ajoute 145 cm3 d'eau et 245 cm3 d'acétate d'éthyle. Le mélange biphasique est refroidi à 0°C sous agitation puis acidifié par de l'acide chlorhydrique 1N jusqu'à pH = 5. La phase aqueuse est séparée par décantation et extraite avec 2 fois 75 cm3 d'acétate d'éthyle. Les phases organiques sont réunies et séchées sur sulfate de sodium. Après filtration et concentration sous pression réduite à 25°C jusqu'à un volume de 50 cm3, on ajoute à cette solution résiduelle, à 0°C, 9,80 g d'acétoxy-4 benzoyloxy-2α époxy-5β,20 dihydroxy-1,13α oxo-9 bis-(trichloro-2,2,2 éthoxy) carbonyloxy-7β,10β taxéne-11, 4,29 g de dicylohexylcarbodiimide et 0,25 g de diméthylamino-4 pyridine. On agite pendant 15 minutes à 0°C puis pendant 3 heures à une température voisine de 20°C. La dicyclohexylurée formé est séparée par filtration et lavée par de l'acétate d'éthyle. Les phases organiques réunies sont lavées par une solution aqueuse saturée de bicarbonate de sodium. Après séchage et concentration à sec sous pression réduite, on obtient 14,75 g de t.butoxycarbonyl-3 méthoxy-2 phényl-4 oxazolidine-1,3 carboxylate-5-(45,5R) d'acétoxy-4 benzoyloxy-2α époxy-5β,20 hydroxy-1 oxo-9 bis(trichloro-2,2,2 éthoxy) carbonyloxy-7β,10β taxène-11 yle-13α, sous la forme d'un mélange diastéréoisomérique, dont les caractéristiques sont les suivantes :
- spectre infra-rouge (CH₂Cl₂) : bandes d'absorption caractéristiques à 1760, 1725-1710, 1600, 1450, 1245, 1175, 1060, 985 et 815 cm⁻¹
- spectre de résonance magnétique nucléaire du proton (400 MHz ; CDCl₃ ; température : 323°K ; déplacements chimiques δ en ppm ; constantes de couplage J en Hz) : 1,23 (s, 3H) ; 1,32 (s, 3H) ; 1,35 (mf, 9H) ; 1,88 (s, 3H) ; 1,91 (s, 3H) ; 2,08 (s,3H) ; 2,08 (mt, 1H); 2,26 (ab dédoublé, J = 15 et 9, 1H); 2,65 (mt, H) ; 3,65 (s, 3H) ; 3,92 (d, J = 7, 1H) ; 4,18 (d, J = 8, 1H) ; 4,31 (d, J = 8, 1H) ; 4,64 (d, J = 12, 1H); 4,80 (d, J = 7, 1H); 4,83 (ab limite, 2H); 4,95 (d large, J = 10, 1H); 4,95 (d, J = 12, 1H); 5,04 (d large, J = 7, 1H); 5,58 (dd, J = 11 et 7, 1H) ; 5,72 (d, J = 7, 1H); 6,25 (s, 1H); 6,31 (s, 1H); 6,34 (t, J = 9, 1H) ; 7,30 à 7,55 (mt, 5H) ; 7,54 (t, J = 7,5, 2H) ; 7,68 (t, J = 7,5, 1H); 8,08 (d, J = 7,5, 2H).

A une solution agitée de 0,617 g d'ester obtenu précédemment dans 7,6 cm3 d'acétate d'éthyle on ajoute 47 µl d'acide chlorhydrique à 37 % (p/p). On agite pendant 20 heures à une température voisine de 20°C. L'analyse par chromatographie liquide à haute performance montre que l'on obtient le t.butoxycarbonylamino3 phényl-3 hydroxy-2 propionate-(2R,3S) d'acétoxy-4 benzoyloxy-2α époxy-5β,20 hydroxy-1 oxo-9 bis-(trichloro-2,2,2 éthoxy)carbonyloxy-7β,10β taxène-11 yle-13α, avec un rendement de 53 %.

### EXEMPLE 10

Une solution de 4,01 g de benzoylamino-3 hydroxy-2 phényl-3 propionate-(2R,3S) de méthyle et de 0,01 g de p.toluènesulfonate de pyridinium dans 70 cm3 de toluène est déshydratée par distillation de 30 cm3 de solvant. On ajoute 30 cm3 de toluène et distille 20 cm3 de solvant. Après refroidissement, on ajoute une solution de 2,57 g de diméthylacétal de p.méthoxybenzaldéhyde dans 6 cm3 de toluène. On ajoute 20 cm3 de toluène puis on chauffe pendant 40 minutes à une température voisine de 100°C tout en distillant 60 cm3 de solvant. Après refroidissement, la solution trouble est filtrée sur coton puis concentrée à sec. On obtient ainsi 6,13 g d'une huile jaunâtre que l'on agite pendant 12 heurse avec 30 cm3 de cyclohexane. Après filtration sur verre fritté et lavage du précipité par 2 fois 10 cm3 de cyclohexane, on obtient, avec un rendement de 91%, 5,09 g de benzoyl-3 (méthoxy-4 phényl)-2 phényl-4 méthoxycarbonyl-5 oxazolidine-1,3-(2R,4S,5R).

A une solution de 4,80 g du produit obtenu précédemment dans 120 cm3 de méthanol, on ajoute 25 cm3 d'une solution aqueuse contenant 834 mg de potasse à 86%. On agite pendant 1 heure à une température voisine de 20°C. Le méthanol est éliminé par distillation sous pression réduite puis on ajoute 25 cm3 d'eau et 50 cm3 d'éther isopropylique. La phase aqueuse est séparée par décantation puis lavée par 2 fois 25 cm3 d'oxyde isopropylique. La phase aqueuse est acidifiée par addition d'acide chlorhydrique concentré jusqu'à pH = 1, puis on ajoute 50 cm3 de dichlorométhane. Après décantation, la phase aqueuse est lavée par 25 cm3 de dichlorométhane. Les phases organiques réunies sont lavées par 25 cm3 d'eau puis séchées sur sulfate de sodium. Après filtration et concentration à sec, on obtient, avec un rendement de 97%, 4,49 g d'acide benzoyl-3 (méthoxy-4 phényl)-2 phényle oxazolidine-1,3 carboxylique -5-(2R,4S,5R).

### EXEMPLE 11

A une solution de 0,137 g de diacétoxy-4,10β benzoyloxy-2α époxy-5β,20 dihydroxy-1,13α oxo-9 triéthylsilyloxy-7β taxène-11 à 85% et de 0,0521 g de dicyclohexylcarbodiimide dans 1 cm3 de toluène, on ajoute une solution de 0,1023 g d'acide benzoyl-3 (méthoxy-4 phényl)-2 phényle oxazolidine-1,3 carboxylique -5-(2R,4S,5R) et de 5,2 mg de diméthylamino-4 pyridine dans 3 cm3 de toluène. On agite pendant 2 heures 15 minutes à une température voisine de 20°C. La dicyclohexylurée est séparée par filtration. On ajoute au filtrat 20 cm3 d'une solution saturée de bicarbonate de sodium. Après décantation la phase aqueuse est extraite par 3 fois 30 cm3 de dichlorométhane. Les phases organiques réunies sont séchées sur sulfate de sodium. Après filtration et concentration, on obtient 0,2108 g d'un produit que l'on purifie par chromatographie sur 7 g de silice contenus dans une colonne de 30 cm de hauteur et de 1,5 cm de diamètre en éluant avec un mélange cyclohexaneacétate d'éthyle (70-30 en volumes). On obtient ainsi, avec un rendement de 70,54%, 127,4 mg de benzoyl-3 (méthoxy-4 phényl)-2 phényl-4 oxazolidine-1,3 carboxylate-5-(2R,4S,5R) de diacétoxy-4,10β benzoyloxy-2α époxy-5β,20 hydroxy-1 oxo-9 triéthylsilyloxy-7β taxène-11 yle-13α dont la structure est confirmée par le spectre de résonance magnétique nucléaire du proton et dont la pureté est voisine de 95%.

A une solution de 40 mg du produit obtenu précédemment dans 2 cm3 d'éthanol, on ajoute 400 µl d'une solution éthanolique d'acide chlorhydrique 0,9 N. On agite pendant 6 heures à une température voisine de 20°C. Un dosage par chromatographie liquide à haute performance montre que le rendement en benzoyl-3 phényl-3 propionate-(2R,3S) de diacétoxy-4,10β benzoyloxy-2α époxy-5β,20 dihydroxy-1,7β oxo-9 taxène-11 yle-13α (ou taxol) est de 51,4%.

## Revendications

1. Procédé de préparation de dérivés du taxane de formule générale : 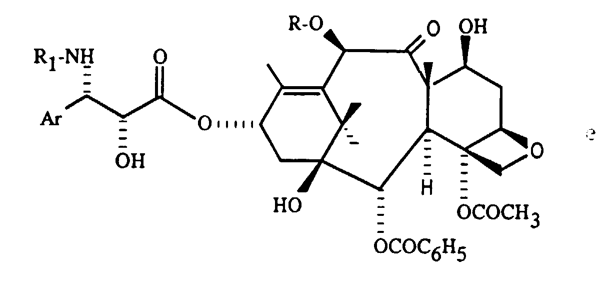 dans laquelle
R représente un atome d'hydrogène ou un radical acétyle et R₁ représente un radical benzoyle ou un radical R₂-O-CO- dans lequel R₂ représente :
- un radical alcoyle droit ou ramifié contenant 1 à 8 atomes de carbone, alcényle contenant 2 à 8 atomes de carbone, alcynyle contenant 3 à 8 atomes de carbone, cycloalcoyle contenant 3 à 6 atomes de carbone, cycloalcényle contenant 4 à 6 atomes de carbone ou bicycloalcoyle contenant 7 à 10 atomes de carbone, ces radicaux étant éventuellement substitués par un ou plusieurs substituants choisis parmi les atomes d'halogène et les radicaux hydroxy, alcoyloxy contenant 1 à 4 atomes de carbone, dialcoylamino dont chaque partie alcoyle contient 1 à 4 atomes de carbone, pipéridino, morpholino, pipérazinyl-1 (éventuellement substitué en -4 par un radical alcoyle contenant 1 à 4 atomes de carbone ou par un radical phénylalcoyle dont la partie alcoyle contient 1 à 4 atomes de carbone), cycloalcoyle contenant 3 à 6 atomes de carbone, cycloalcényle contenant 4 à 6 atomes de carbone, phényle, cyano, carboxy ou alcoyloxycarbonyle dont la partie alcoyle contient 1 à 4 atomes de carbone,
- ou un radical phényle éventuellement substitué par un ou plusieurs atomes ou radicaux choisis parmi les radicaux alcoyles contenant 1 à 4 atomes de carbone ou alcoyloxy contenant 1 à 4 atomes de carbone,
- ou un radical hétérocyclyle azoté saturé ou non saturé contenant 5 ou 6 chaînons éventuellement substitué par un ou plusieurs radicaux alcoyles contenant 1 à 4 atomes de carbone,
étant entendu que les radicaux cycloalcoyles, cycloalcényles ou bicycloalcoyles peuvent être éventuellement substitués par un ou plusieurs radicaux alcoyles contenant 1 à 4 atomes de carbone, et
Ar représente un radical phényle ou α- ou β-naphtyle éventuellement substitué par un ou plusieurs atomes ou radicaux choisis parmi les atomes d'halogène (fluor, chlore, brome, iode) et les radicaux alcoyles, alcényles, alcynyles, aryles, arylalcoyles, alcoxy, alcoylthio, aryloxy, arylthio, hydroxy, hydroxyalcoyle, mercapto, formyle, acyle, acylamino, aroylamino, alcoxycarbonylamino, amino, alkylamino, diakylamino, carboxy, alcoxycarbonyle, carbamoyle, dialcoylcarbamoyle, cyano et trifluorométhyle, étant entendu que les radicaux alcoyles et les portions alcoyles des autres radicaux contiennent 1 à 4 atomes de carbone, que les radicaux alcényles et alcynyles contiennent 3 à 8 atomes de carbone et les radicaux aryles sont les radicaux phényles ou α- ou β-naphtyles
caractérisé en ce que l'on estérifie un dérivé de la baccatine III ou de la désacétyl-10 baccatine III protégée de formule générale : 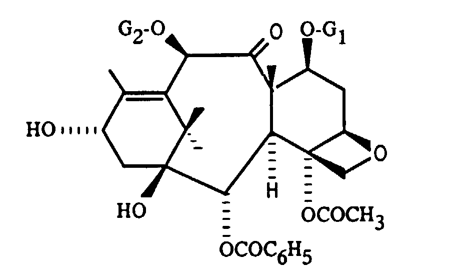 dans laquelle G₁ représente un groupement protecteur de la fonction hydroxy et G₂ représente un radical acétyle ou un groupement protecteur de la fonction hydroxy, au moyen d'un acide de formule générale : 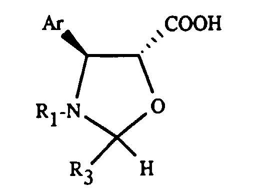 dans laquelle Ar et R₁ sont définis comme précédemment et R₃ représente un atome d'hydrogène ou un radical aryle éventuellement substitué, ou d'un dérivé de cet acide pour obtenir un produit de formule générale : 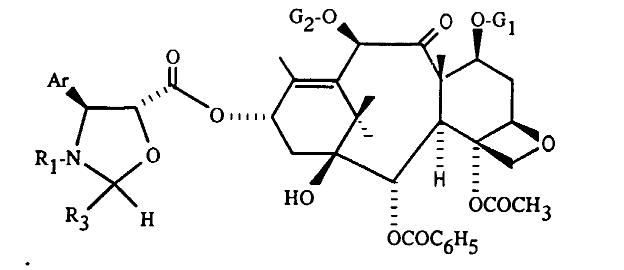 dans laquelle Ar, R₁, R₃, G₁ et G₂ sont définis comme précédemment, dont on déprotège la chaîne latérale et éventuellement les fonctions hydroxy protégées par G₁ et G₂ pour obtenir un produit de formule générale : 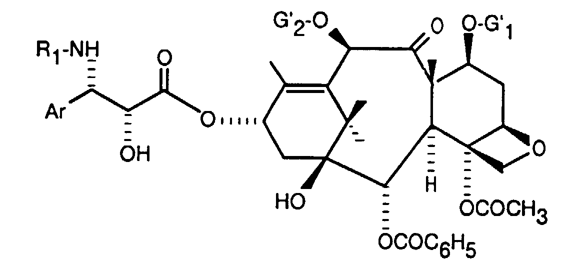 dans laquelle Ar et R₁ sont définis comme précédemment, G'₁ représente un atome d'hydrogène ou un groupement protecteur de la fonction hydroxy et G'₂ représente un atome d'hydrogène ou un radical acétyle ou un groupement protecteur de la fonction hydroxy dont on remplace éventuellement les groupements protecteurs G'₁ et éventuellement G'₂ par des atomes d'hydrogène selon des méthodes connues.

2. Procédé selon la revendication 1 caractérisé en ce que l'on effectue l'estérification au moyen d'un acide, ou d'un de ses dérivés, pour lequel Ar et R₁ étant définis comme dans la revendication 1, R₃ représente un atome d'hydrogène ou un radical alcoxy contenant 1 à 4 atomes de carbone ou un radical phényle éventuellement substitué par un ou plusieurs radicaux électro-donneurs.

3. Procédé selon la revendication 2 caractérisé en ce que les radicaux électro-donneurs sont choisis parmi les radicaux alcoxy contenant 1 à 4 atomes de carbone.

4. Procédé selon la revendication 1 caractérisé en ce que les groupements protecteurs de la baccatine III ou de la désacétyl-10 baccatine III représentés par G₁ et G₂ sont choisis parmi les radicaux (trichloro-2,2,2 éthoxy) carbonyle et (trichlorométhyl-2 propoxy)-2 carbonyle et les radicaux trialkylsilyle, dialkylarylsilyle, alkyldiarylsilyle ou triarylsilyle dans lesquels les parties alkyles contiennent 1 à 4 atomes de carbone et les parties aryles sont de préférence des radicaux phényles.

5. Procédé selon l'une des revendications 1 à 4 caractérisé en ce que l'estérification au moyen d'un acide de formule générale : 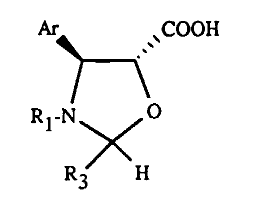 dans laquelle Ar et R₁ sont définis comme dans la revendication 1 et R₃ est défini comme dans l'une des revendications 1 à 3 est effectuée en présence d'un agent de condensation et d'un agent d'activation en opérant dans un solvant organique à une température comprise entre -10 et 90°C.

6. Procédé selon la revendication 5 caractérisé en ce que l'agent de condensation est choisi parmi les carbodiimides et les carbonates réactifs et l'agent d'activation est choisi parmi les aminopyridines.

7. Procédé selon la revendication 6 caractérisé en ce que l'agent de condensation est choisi parmi la dicyclohexylcarbodiimide et le dipyridyl-2 carbonate et l'agent d'activation est choisi parmi la diméthylamino-4 pyridine et le pyrrolidino-4 pyridine.

8. Procédé selon la revendication 5 caractérisé en ce que le solvant est choisi parmi les éthers, les cétones, les esters, les nitriles, les hydrocarbures aliphatiques, les hydrocarbures aliphatiques halogénés et les hydrocarbures aromatiques.

9. Procédé selon la revendication 8 caractérisé en ce que le solvant est choisi parmi les hydrocarbures aromatiques.

10. Procédé selon l'une des revendications 1 à 4 caractérisé en ce que l'estérification est effectuée au moyen d'un anhydride de formule générale : 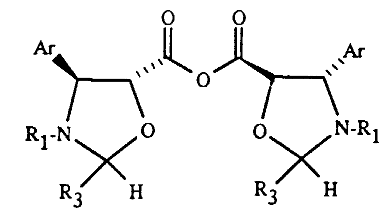 dans laquelle Ar et R₁ sont définis comme dans la revendication 1 et R₃ est défini comme dans l'une des revendications 1 à 3 en opérant en présence d'un agent d'activation dans un solvant organique à une température comprise entre 0 et 90°C.

11. Procédé selon la revendication 10 caractérisé en ce que l'agent d'activation est choisi parmi les aminopyridines.

12. Procédé selon la revendication 11 caractérisé en ce que l'agent d'activation est choisi parmi la diméthylamino-4 pyridine et la pyrrolidino-4 pyridine.

13. Procédé selon la revendication 10 caractérisé en ce que le solvant est choisi parmi les éthers, les cétones, les esters, les nitriles, les hydrocarbures aliphatiques, les hydrocarbures aliphatiques halogénés et les hydrocarbures aromatiques.

14. Procédé selon l'une des revendications 1 à 4 caractérisé en ce que l'estérification est effectuée au moyen d'un acide activé de formule : 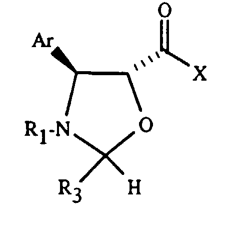 dans laquelle Ar et R₁ sont définis comme dans la revendication 1, R₃ est défini comme dans l'une des revendications 1 à 3 et X représente un atome d'halogène ou un radical acyloxy ou aroyloxy, éventuellement préparé in situ, en présence d'une base dans un solvant organique à une température comprise entre 10 et 80°C.

15. Procédé selon la revendication 14 caractérisé en ce que la base est choisie parmi les bases organiques azotées.

16. Procédé selon la revendication 15 caractérisé en ce que la base organique azotée est choisie parmi les amines tertiaires aliphatiques, la pyridine et les aminopyridines.

17. Procédé selon la revendication 14 caractérisé en ce que le solvant est choisi parmi les éthers, les cétones, les esters, les nitriles, les hydrocarbures aliphatiques, les hydrocarbures aliphatiques halogénés et les hydrocarbures aromatiques.

18. Procédé selon la revendication 17 caractérisé en ce que le solvant est choisi parmi les hydrocarbures aromatiques.

19. Procédé selon l'une des revendications 1 à 4 caractérisé en ce que la déprotection de la chaîne latérale et éventuellement des fonctions hydroxy protégées par des groupements protecteurs G₁ et G₂ silylés est effectuée en présence d'un acide minéral ou organique ou de leurs mélanges en opérant dans un solvant organique à une température comprise entre -10 et 60°C.

20. Procédé selon la revendication 19 caractérisé en ce que l'acide minéral est choisi parmi les acides chlorhydrique et sulfurique et l'acide organique est choisi parmi les acides acétique, méthanesulfonique, trifluorométhanesulfonique et p.toluènesulfonique.

21. Procédé selon la revendication 19 caractérisé en ce que le solvant est choisi parmi les alcools, les éthers, les esters, les nitriles, les hydrocarbures aliphatiques, les hydrocarbures aliphatiques halogénés et les hydrocarbures aromatiques.

22. Procédé selon la revendication 1 caractérisé en ce que la déprotection de la chaîne latérale est effectuée en présence d'un oxydant dans l'eau ou en milieu hydro-organique.

23. Procédé selon la revendication 22 caractérisé en ce que l'oxydant est le nitrate d'ammonium et de cérium IV en milieu hydro-organique.

24. Procédé selon l'une des revendications 22 ou 23 caractérisé en ce que le milieu hydro-organique est un mélange eau-acétonitrile.

25. Procédé selon la revendication 22 caractérisé en ce que l'oxydant est la dichloro-2,3 dicyano-5,6 benzoquinone-1,4 dans l'eau.

26. Procédé selon la revendication 1 caractérisé en ce que la déprotection de la chaîne latérale est effectuée par hydrogénolyse.

27. Procédé selon la revendication 26 caractérisé en ce que l'hydrogénolyse est effectuée par l'hydrogène en présence d'un catalyseur.

28. Procédé selon la revendication 1 caractérisé en ce que le remplacement des groupements protecteurs G₁ et éventuellement G₂ représentant un radical trichloro-2,2,2 éthoxycarbonyle ou (trichlorométhyl-2 propoxy)-2 carbonyle par des atomes d'hydrogène est effectué par le zinc, éventuellement associé à du cuivre, en présence d'acide acétique à une température comprise entre 20 et 60°C ou au moyen d'un acide minéral ou organique en solution dans un alcool aliphatique contenant 1 à 3 atomes de carbone ou dans un ester aliphatique en présence de zinc éventuellement associé à du cuivre.

29. Les acides de formule générale : 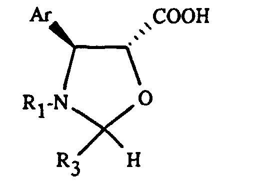 dans laquelle Ar et R₁ sont définis comme dans la revendication 1 et R₃ est défini comme dans l'une des revendications 1 à 3, éventuellement sous forme de sel, d'ester, d'anhydride, d'anhydride mixte ou d'halogénure.

30. Un produit de formule générale : 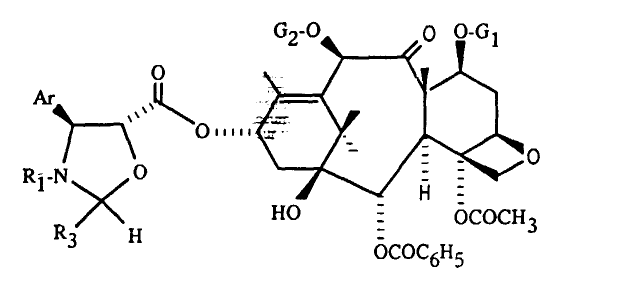 dans laquelle Ar et R₁ sont définis comme dans la revendication 1, R₃ est défini comme dans l'une des revendications 1 à 3 et G₁ et G₂ sont définis comme dans l'une des revendications 1 ou 4.

## Patentansprüche

1. Verfahren zur Herstellung von Taxanderivaten der allgemeinen Formel 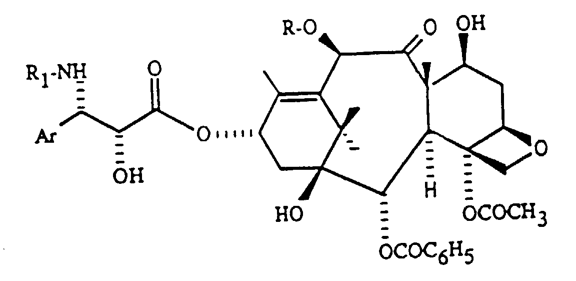 worin
R ein Wasserstoffatom oder einen Acetylrest bedeutet und R₁ einen Benzoylrest oder einen Rest R₂-O-CO- wiedergibt, worin R₂ darstellt:
- einen geradkettigen oder verzweigten Alkylrest mit 1 bis 8 Kohlenstoffatomen, Alkenyl mit 2 bis 8 Kohlenstoffatomen, Alkinyl mit 3 bis 8 Kohlenstoffatomen, Cycloalkyl mit 3 bis 6 Kohlenstoffatomen, Cycloalkenyl mit 4 bis 6 Kohlenstoffatomen oder Bicycloalkyl mit 7 bis 10 Kohlenstoffatomen, wobei diese Reste gegebenenfalls substituiert sind durch einen oder mehrere Substituenten, ausgewählt unter den Halogenatomen und den Hydroxygruppen, Alkoxy mit 1 bis 4 Kohlenstoffatomen, Dialkylamino, dessen Alkylteil bis 4 Kohlenstoffatome enthält, Piperidino, Morpholino, 1-Piperazinyl (gegebenenfalls substituiert in 4-Stellung mit einem Alkylrest mit 1 bis 4 Kohlenstoffatomen oder mit einem Phenylalkylrest, dessen Alkylteil 1 bis 4 Kohlenstoffatome enthält), Cycloalkyl mit 3 bis 6 Kohlenstoffatomen, Cycloalkenyl mit 4 bis 6 Kohlenstoffatomen, Phenyl, Cyano, Carboxy oder Alkoxycarbonyl, dessen Alkylteil 1 bis 4 Kohlenstoffatome enthält,
- oder einen Phenylrest, gegebenenfalls substituiert durch ein oder mehrere Atome oder Reste, ausgewählt unter den Alkylresten mit 1 bis 4 Kohlenstoffatomen oder Alkoxyresten mit 1 bis 4 Kohlenstoffatomen,
- oder einen stickstoffhaltigen, gesättigten oder ungesättigten Heterocyclus mit 5 oder 6 Gliedern, gegebenenfalls substituiert durch einen oder mehrere Alkylreste mit 1 bis 4 Kohlenstoffatomen,
mit der Maßgabe, daß die Cycloalkyl-, Cycloalkenyl- oder Bicycloalkylreste gegebenenfalls durch einen oder mehrere Alkylreste mit 1 bis 4 Kohlenstoffatomen substituiert sein können und
Ar einen Phenyl- oder α- oder β-Naphthylrest, gegebenenfalls substituiert durch einen oder mehrere Atome oder Reste, ausgewählt unter den Halogenatomen (Fluor, Chlor, Brom, Jod) und den Alkyl-, Alkenyl-, Alkinyl-, Aryl-, Aralkyl-, Alkoxy-, Alkylthio-, Aryloxy-, Arylthio-, Hydroxy-, Hydroxyalkyl-, Mercapto-, Formyl-, Acyl-, Acylamino-, Aroylamino-, Alkoxycarbonylamino-, Amino-, Alkylamino-, Dialkylamino-, Carboxy-, Alkoxycarbonyl-, Carbamoyl-, Dialkylcarbamoyl-, Cyano- und Trifluormethylgruppen, bedeutet, mit der Maßgabe, daß die Alkylreste und die Alkylteile der anderen Reste 1 bis 4 Kohlenstoffatome aufweisen, daß die Alkenylreste und die Alkinylreste 3 bis 8 Kohlenstoffatome aufweisen und die Arylreste Phenyl- oder α- oder ß-Naphthylreste sind, dadurch gekennzeichnet, daß man ein geschütztes Derivat von Baccatin III oder 10-Desacetyl-baccatin III der allgemeinen Formel 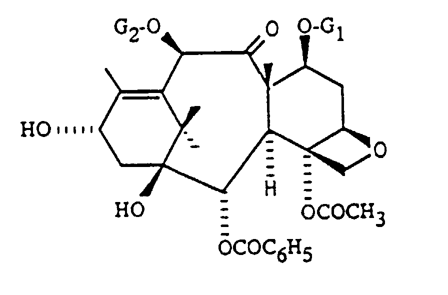 worin G₁ eine Schutzgruppe für die Hydroxyfunktion bedeutet und G₂ einen Acetylrest oder eine Schutzgruppe für die Hydroxyfunktion bedeutet, mit Hilfe einer Säure der allgemeinen Formel 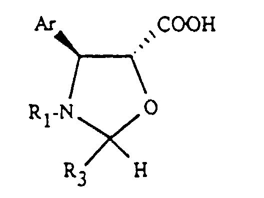 worin Ar und R₁ wie vorstehend definiert sind und R₃ ein Wasserstoffatom oder einen gegebenenfalls substituierten Arylrest bedeutet, oder mit Hilfe eines Derivats dieser Säure verestert, um zu einem Produkt der allgemeinen Formel 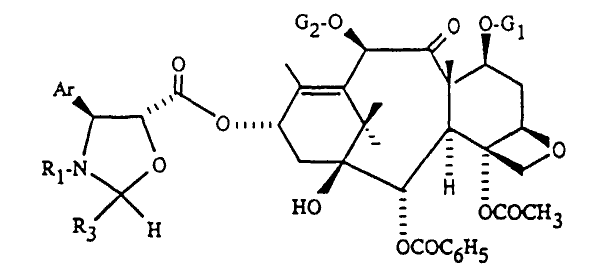 zu gelangen, worin Ar, R₁, R₃, G₁ und G₂ wie vorstehend definiert sind, an dem man gegebenenfalls die Schutzgruppe der Seitenkette und gegebenenfalls die Schutzgruppe der durch G₁ und G₂ geschützten Hydroxyfunktionen entfernt, um zu einem Produkt der allgemeinen Formel 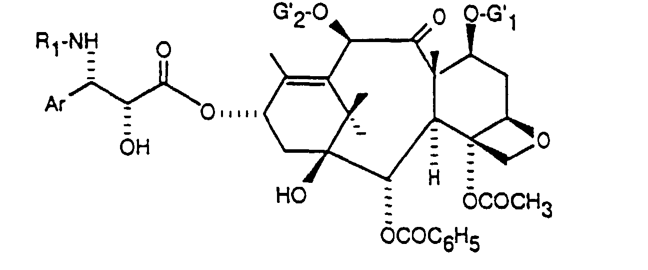 zu gelangen, worin Ar und R₁ wie vorstehend definiert sind, G'₁ ein Wasserstoffatom oder eine Schutzgruppe der Hydroxyfunktion bedeutet und G'₂ ein Wasserstoffatom oder einen Acetylrest oder eine Schutzgruppe der Hydroxyfunktion bedeutet, an welchem Produkt man gegebenenfalls die Schutzgruppen G'₁ und gegebenenfalls G'₂ durch Wasserstoffatome nach bekannten Methoden ersetzt.

2. Verfahren gemäß Anspruch 1, dadurch gekennzeichnet, daß man die Veresterung mit Hilfe einer Säure oder eines ihrer Derivate durchführt, worin Ar und R₁ wie in Anspruch 1 definiert sind, R₃ ein Wasserstoffatom oder einen Alkoxyrest mit 1 bis 4 Kohlenstoffatomen oder einen gegebenenfalls durch einen oder mehrere Elektronen-Donor-Reste substituierten Phenylrest wiedergibt.

3. Verfahren gemäß Anspruch 2, dadurch gekennzeichnet, daß die Elektronen-Donor-Reste unter den Alkoxyresten mit 1 bis 4 Kohlenstoffatomen ausgewählt werden.

4. Verfahren gemäß Anspruch 1, dadurch gekennzeichnet, daß die Schutzgruppen von Baccatin III oder von 10-Desacetyl-baccatin III, die durch G₁ und G₂ dargestellt sind, unter den (2,2,2-Trichlorethoxy)-carbonyl- und 2-(2-Trichlormethylpropoxy)-carbonylresten und den Trialkylsilyl-, Dialkylarylsilyl-, Alkyldiarylsilyl- oder Triarylsilylresten ausgewählt werden, worin die Alkylteile 1 bis 4 Kohlenstoffatome aufweisen und die Arylteile bevorzugt Phenylreste sind.

5. Verfahren gemäß einem der Ansprüche 1 bis 4, dadurch gekennzeichnet, daß die Veresterung mit einer Säure der allgemeinen Formel 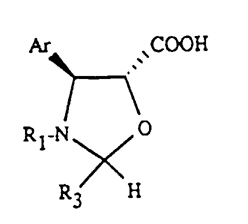 worin Ar und R₁ wie in Anspruch 1 definiert sind und R₃ wie in einem der Ansprüche 1 bis 3 definiert ist, in Gegenwart eines Kondensationsmittels und eines Aktivierungsmittels durchgeführt wird, wobei man in einem organischen Lösungsmittel bei einer Temperatur zwischen -10 und 90°C arbeitet.

6. Verfahren gemäß Anspruch 5, dadurch gekennzeichnet, daß das Kondensationsmittel unter den Carbodiimiden und den reaktiven Carbonaten ausgewählt wird und das Aktivierungsmittel unter den Aminopyridinen ausgewählt wird.

7. Verfahren gemäß Anspruch 6, dadurch gekennzeichnet, daß das Kondensationsmittel unter Dicyclohexylcarbodiimid und Dipyrid-2-yl-carbonat ausgewählt wird und das Aktivierungsmittel unter 4-Dimethylaminopyridin und 4-Pyrrolidinopyridin ausgewählt wird.

8. Verfahren gemäß Anspruch 5, dadurch gekennzeichnet, daß das Lösungsmitteln unter den Ethern, den Ketonen, den Estern, den Nitrilen, den aliphatischen Kohlenwasserstoffen, den halogenierten, aliphatischen Kohlenwasserstoffen und den aromatischen Kohlenwasserstoffen ausgewählt wird.

9. Verfahren gemäß Anspruch 8, dadurch gekennzeichnet, daß das Lösungsmittel unter den aromatischen Kohlenwasserstoffen ausgewählt wird.

10. Verfahren gemäß einem der Ansprüche 1 bis 4, dadurch gekennzeichnet, daß die Veresterung mit Hilfe eines Anhydrids der allgemeinen Formel 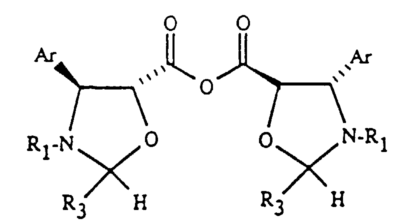 worin Ar und R₁ wie in Anspruch 1 definiert sind und R₃ wie in einem der Ansprüche 1 bis 3 definiert ist, durchgeführt wird, indem man in Gegenwart eines Aktivierungsmittels in einem organischen Lösungsmittel bei einer Temperatur zwischen 0 und 90°C arbeitet.

11. Verfahren gemäß Anspruch 10,dadurch gekennzeichnet, daß das Aktivierungsmittel unter den Aminopyridinen ausgewählt wird.

12. Verfahren gemäß Anspruch 11, dadurch gekennzeichnet, daß das Aktivierungsmittel unter 4-Dimethylaminopyridin und 4-Pyrrolidinopyridin ausgewählt wird.

13. Verfahren gemäß Anspruch 10, dadurch gekennzeichnet, daß das Lösungsmittel unter den Ethern, den Ketonen, den Estern, den Nitrilen, den aliphatischen Kohlenwasserstoffen, den halogenierten, aliphatischen Kohlenwasserstoffen und den aromatischen Kohlenwasserstoffen ausgewählt wird.

14. Verfahren gemäß einem der Ansprüche 1 bis 4, dadurch gekennzeichnet, daß die Veresterung mit Hilfe einer aktivierten Säure der Formel 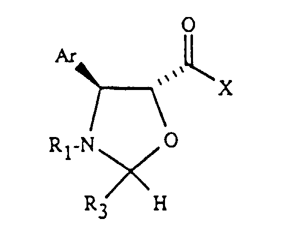 worin Ar und R₁ wie in Anspruch 1 definiert sind, R₃ wie in einem der Ansprüche 1 bis 3 definiert ist und X für ein Halogenatom oder einen Acyloxy- oder Aroyloxyrest steht, welche gegebenenfalls in situ hergestellt wurde, in Anwesenheit einer Base in einem organischen Lösungsmittel bei einer Temperatur zwischen 10 und 80°C durchgeführt wird.

15. Verfahren gemäß Anspruch 14,dadurch gekennzeichnet, daß die Base unter den organischen Stickstoffbasen ausgewählt wird.

16. Verfahren gemäß Anspruch 15,dadurch gekennzeichnet, daß die organische Stickstoffbase unter den aliphatischen, tertiären Aminen, Pyridin und den Aminopyridinen ausgewählt wird.

17. Verfahren gemäß Anspruch 14,dadurch gekennzeichnet, daß das Lösungsmittel unter den Ethern, den Ketonen, den Estern, den Nitrilen, den aliphatischen Kohlenwasserstoffen, den halogenierten, aliphatischen Kohlenwasserstoffen und den aromatischen Kohlenwasserstoffen ausgewählt wird.

18. Verfahren gemäß Anspruch 17,dadurch gekennzeichnet, daß das Lösungsmittel unter den aromatischen Kohlenwasserstoffen ausgewählt wird.

19. Verfahren gemäß einem der Ansprüche 1 bis 4, dadurch gekennzeichnet, daß die Abspaltung der Schutzgruppe von der Seitenkette und gegebenenfalls von den durch die silylierten Schutzgruppen G₁ und G₂ geschützten Hydroxyfunktionen in Anwesenheit einer Mineral- oder organischen Säure oder von deren Gemischen erfolgt, wobei man in einem organischen Lösungsmittel bei einer Temperatur zwischen -10 und 60°C arbeitet.

20. Verfahren gemäß Anspruch 19,dadurch gekennzeichnet, daß die Mineralsäure unter Chlorwasserstoffsäure und Schwefelsäure ausgewählt wird und die organische Säure unter Essigsäure, Methansulfonsäure, Trifluormethansulfonsäure und p-Toluolsulfonsäure ausgewählt wird.

21. Verfahren gemäß Anspruch 19,dadurch gekennzeichnet, daß das Lösungsmittel unter den Alkoholen, den Ethern, den Estern, den Nitrilen, den aliphatischen Kohlenwasserstoffen, den halogenierten, aliphatischen Kohlenwasserstoffen und den aromatischen Kohlenwasserstoffen ausgewählt wird.

22. Verfahren gemäß Anspruch 1, dadurch gekennzeichnet, daß die Abspaltung der Schutzgruppe von der Seitenkette in Anwesenheit eines Oxidationsmittels in Wasser oder in einem wäßrig-organischen Milieu erfolgt.

23. Verfahren gemäß Anspruch 22,dadurch gekennzeichnet, daß das Oxidationsmittel Ammonium- und Cer(IV)-nitrat in wäßrig-organischem Milieu ist.

24. Verfahren gemäß einem der Ansprüche 22 oder 23, dadurch gekennzeichnet, daß das wäßrig-organische Milieu ein Wasser-Acetonitril-Gemisch ist.

25. Verfahren gemäß Anspruch 22,dadurch gekennzeichnet, daß das Oxidationsmittel 2,3-Dichlor-5,6-dicyano-1,4-benzochinon in Wasser ist.

26. Verfahren gemäß Anspruch 1, dadurch gekennzeichnet, daß die Abspaltung der Schutzgruppe von der Seitenkette durch Hydrogenolyse erfolgt.

27. Verfahren gemäß Anspruch 26, dadurch gekennzeichnet, daß die Hydrogenolyse mit Wasserstoff in Anwesenheit eines Katalysators durchgeführt wird.

28. Verfahren gemäß Anspruch 1, dadurch gekennzeichnet, daß der Austausch der Schutzgruppen G₁ und gegebenenfalls G₂, welche einen 2,2,2-Trichlorethoxycarbonyl- oder 2-(2-Trichlormethylpropoxy)-carbonylrest darstellen, mit Wasserstoffatomen durch Zink, gegebenenfalls gemeinsam mit Kupfer, in Anwesenheit von Essigsäure bei einer Temperatur zwischen 20 und 60°C oder mit Hilfe einer Mineral- oder organischen Säure in Lösung in einem aliphatischen Alkohol mit 1 bis 3 Kohlenstoffatomen oder in einem aliphatischen Ester in Anwesenheit von Zink, gegebenenfalls gemeinsam mit Kupfer, durchgeführt wird.

29. Die Säuren der allgemeinen Formel 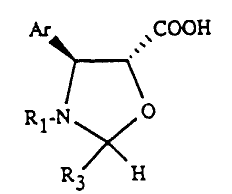 worin Ar und R₁ wie in Anspruch 1 definiert sind und R₃ wie in einem der Ansprüche 1 bis 3 definiert ist, gegebenfalls in Form des Salzes, Esters, Anhydrids, gemischten Anhydrids oder Halogenids.

30. Produkt der allgemeinen Formel 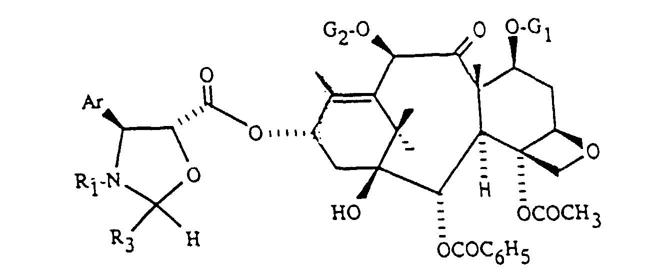 worin Ar und R₁ wie in Anspruch 1 definiert sind, R₃ wie in einem der Ansprüche bis 3 definiert ist und G₁ und G₂ wie in einem der Ansprüche 1 oder 4 definiert sind.

## Claims

1. Process for preparing taxane derivatives of general formula: 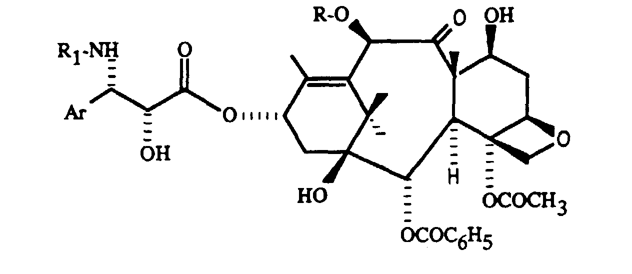 in which
R represents a hydrogen atom or an acetyl radical, and R₁ represents a benzoyl radical or a radical R₂-O-CO- in which R₂ represents:
- an unbranched or branched alkyl radical containing 1 to 8 carbon atoms, an alkenyl radical containing 2 to 8 carbon atoms, an alkynyl radical containing 3 to 8 carbon atoms, a cycloalkyl radical containing 3 to 6 carbon atoms, a cycloalkenyl radical containing 4 to 6 carbon atoms or a bicycloalkyl radical containing 7 to 10 carbon atoms, these radicals being optionally substituted with one or more substituents chosen from halogen atoms and hydroxyl radicals, alkyloxy radicals containing 1 to 4 carbon atoms, dialkylamino radicals in which each alkyl portion contains 1 to 4 carbon atoms, piperidino radicals, morpholino radicals, 1-piperazinyl radicals (optionally substituted at position 4 with an alkyl radical containing 1 to 4 carbon atoms or with a phenylalkyl radical in which the alkyl portion contains 1 to 4 carbon atoms), cycloalkyl radicals containing 3 to 6 carbon atoms, cycloalkenyl radicals containing 4 to 6 carbon atoms, phenyl radicals, cyano radicals, carboxyl radicals or alkyloxycarbonyl radicals in which the alkyl portion contains 1 to 4 carbon atoms,
- or a phenyl radical optionally substituted with one or more atoms or radicals chosen from alkyl radicals containing 1 to 4 carbon atoms or alkyloxy radicals containing 1 to 4 carbon atoms,
- or a saturated or unsaturated 5- or 6-membered nitrogenous heterocyclic radical optionally substituted with one or more alkyl radicals containing 1 to 4 carbon atoms,
on the understanding that the cycloalkyl, cycloalkenyl or bicycloalkyl radicals can be optionally substituted with one or more alkyl radicals containing 1 to 4 carbon atoms, and
Ar represents a phenyl or α- or β-naphthyl radical optionally substituted with one or more atoms or radicals chosen from halogen (fluorine, chlorine, bromine, iodine) atoms and alkyl, alkenyl, alkynyl, aryl, arylalkyl, alkoxy, alkylthio, aryloxy, arylthio, hydroxyl, hydroxyalkyl, mercapto, formyl, acyl, acylamino, aroylamino, alkoxycarbonylamino, amino, alkylamino, dialkylamino, carboxyl, alkoxycarbonyl, carbamoyl, dialkylcarbamoyl, cyano and trifluoromethyl radicals, on the understanding that the alkyl radicals and alkyl portions of the other radicals contain 1 to 4 carbon atoms, and that the alkenyl and alkynyl radicals contain 3 to 8 carbon atoms and the aryl radicals are phenyl or α- or β-naphthyl radicals
characterized in that a protected 10-deacetylbaccatin III or baccatin III derivative of general formula: 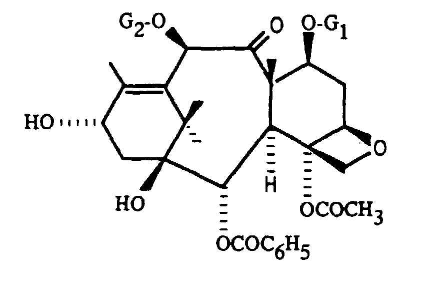 in which G₁ represents a group protecting the hydroxyl function and G₂ represents an acetyl radical or a group protecting the hydroxyl function, is esterified by means of an acid of general formula: 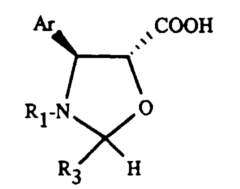 in which Ar and R₁ are defined as above and R₃ represents a hydrogen atom or an optionally substituted aryl radical, or of a derivative of this acid, to obtain a product of general formula: 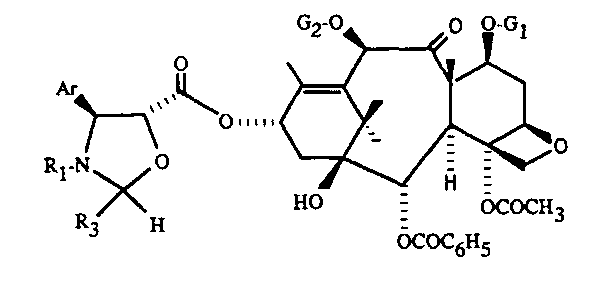 in which Ar, R₁, R₃, G₁ and G₂ are defined as above, the side chain of which and, where appropriate, the hydroxyl functions of which, protected by G₁ and G₂, is/are deprotected to obtain a product of general formula: 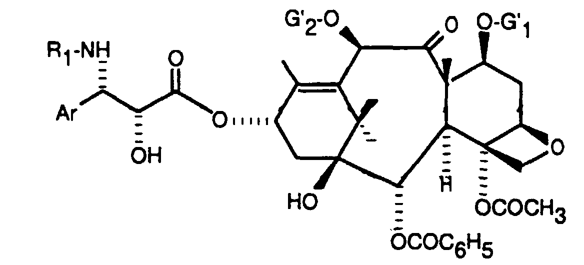 in which Ar and R₁ are defined as above, G'₁ represents a hydrogen atom or a group protecting the hydroxyl function and G'₂ represents a hydrogen atom or an acetyl radical or a group protecting the hydroxyl function, the protective groups G'₁ and, where appropriate, G'₂ of which are replaced, where appropriate, by hydrogen atoms according to known methods.

2. Process according to claim 1, characterized in that the esterification is performed by means of an acid or one of its derivatives for which, Ar and R₁ being defined as in claim 1, R₃ represents a hydrogen atom or an alkoxy radical containing 1 to 4 carbon atoms or a phenyl radical optionally substituted with one or more electron-donating radicals.

3. Process according to claim 2, characterized in that the electron-donating radicals are chosen from alkoxy radicals containing 1 to 4 carbon atoms.

4. Process according to claim 1, characterized in that the groups protecting baccatin III or 10-deacetylbaccatin III represented by G₁ and G₂ are chosen from (2,2,2-trichloroethoxy)carbonyl and (2-trichloromethyl-2-propoxy)carbonyl radicals and trialkylsilyl, dialkylarylsilyl, alkyldiarylsilyl or triarylsilyl radicals in which the alkyl portions contain 1 to 4 carbon atoms and the aryl portions are preferably phenyl radicals.

5. Process according to one of claims 1 to 4, characterized in that the esterification by means of an acid of general formula: 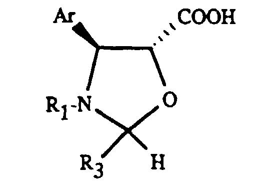 in which Ar and R₁ are defined as in claim 1 and R₃ is defined as in one of claims 1 to 3, is performed in the presence of a condensing agent and an activating agent, working in an organic solvent at a temperature of between -10 and 90°C.

6. Process according to claim 5, characterized in that the condensing agent is chosen from carbodiimides and reactive carbonates and the activating agent is chosen from aminopyridines.

7. Process according to claim 6, characterized in that the condensing agent is chosen from dicyclohexylcarbodiimide and di-2-pyridyl carbonate and the activating agent is chosen from 4-(dimethylamino)pyridine and 4-pyrrolidinopyridine.

8. Process according to claim 5, characterized in that the solvent is chosen from ethers, ketones, esters, nitriles, aliphatic hydrocarbons, halogenated aliphatic hydrocabons and aromatic hydrocarbons.

9. Process according to claim 8, characterized in that the solvent is chosen from aromatic hydrocarbons.

10. Process according to one of claims 1 to 4, characterized in that the esterification is performed by means of an anhydride of general formula: 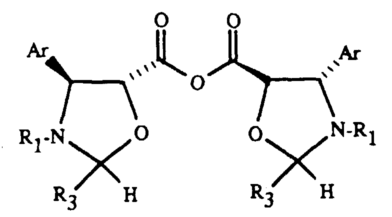 in which Ar and R₁ are defined as in claim 1 and R₃ is defined as in one of claims 1 to 3, working in the presence of an activating agent in an organic solvent at a temperature of between 0 and 90°C.

11. Process according to claim 10, characterized in that the activating agent is chosen from aminopyridines.

12. Process according to claim 11, characterized in that the activating agent is chosen from 4-(dimethylamino)pyridine and 4-pyrrolidinopyridine.

13. Process according to claim 10, characterized in that the solvent is chosen from ethers, ketones, esters, nitriles, aliphatic hydrocarbons, halogenated aliphatic hydrocarbons and aromatic hydrocarbons.

14. Process according to one of claims 1 to 4, characterized in that the esterification is performed by means of an activated acid of formula: 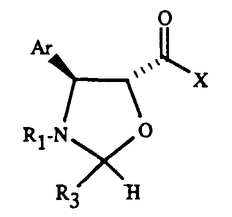 in which Ar and R₁ are defined as in claim 1, R₃ is defined as in one of claims 1 to 3 and X represents a halogen atom or an acyloxy or aryloxy radical, optionally prepared in situ, in the presence of a base in an organic solvent at a temperature of between 10 and 80°C.

15. Process according to claim 14, characterized in that the base is chosen from nitrogenous organic bases.

16. Process according to claim 15, characterized in that the nitrogenous organic base is chosen from aliphatic tertiary amines, pyridine and aminopyridines.

17. Process according to claim 14, characterized in that the solvent is chosen from ethers, ketones, esters, nitriles, aliphatic hydrocarbons, halogenated aliphatic hydrocarbons and aromatic hydrocarbons.

18. Process according to claim 17, characterized in that the solvent is chosen from aromatic hydrocarbons.

19. Process according to one of claims 1 to 4, characterized in that the deprotection of the side chain and, where appropriate, of the hydroxyl functions protected by silyl protective groups G₁ and G₂ is performed in the presence of an inorganic or organic acid or mixtures thereof, working in an organic solvent at a temperature of between -10 and 60°C.

20. Process according to claim 19, characterized in that the inorganic acid is chosen from hydrochloric and sulphuric acids and the organic acid is chosen from acetic, methanesulphonic, trifluoromethanesulphonic and p-toluenesulphonic acids.

21. Process according to claim 19, characterized in that the solvent is chosen from alcohols, ethers, esters, nitriles, aliphatic hydrocarbons, halogenated aliphatic hydrocarbons and aromatic hydrocarbons.

22. Process according to claim 1, characterized in that the deprotection of the side chain is performed in the presence of an oxidizing agent in water or in an aqueous-organic medium.

23. Process according to claim 22, characterized in that the oxdizing agent is ammonium cerium IV nitrate in an aqueous-organic medium.

24. Process according to one of claims 22 and 23, characterized in that the aqueous-organic medium is a water/acetonitrile mixture.

25. Process according claim 22, characterized in that the oxidizing agent is 2,3-dichloro-5,6-dicyano-1,4-benzoquinone in water.

26. Process according to claim 1, characterized in that the deprotection of the side chain is performed by hydrogenolysis.

27. Process according to claim 26, characterized in that the hydrogenolysis is performed with hydrogen in the presence of a catalyst.

28. Process according to claim 1, characterized in that replacement of the protective groups G₁ and, where appropriate, G₂ representing a 2,2,2-trichloroethoxycarbonyl or (2-trichloromethyl-2-propoxy)carbonyl radical by hydrogen atoms is performed with zinc, optionally in combination with copper, in the presence of acetic acid at a temperature of between 20 and 60°C, or by means of an inorganic or organic acid dissolved in an aliphatic alcohol containing 1 to 3 carbon atoms or in an aliphatic ester, in the presence of zinc optionally in combination with copper.

29. The acids of general formula: 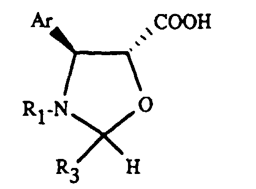 in which Ar and R₁ are defined as in claim 1 and R₃ is defined as in one of claims 1 to 3, optionally in the form of a salt, ester, anhydride, mixed anhydride or halide.

30. A product of general formula: 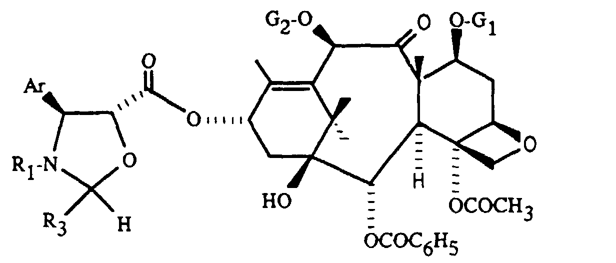 in which Ar and R₁ are defined as in claim 1, R₃ is defined as in one of claims 1 to 3 and G₁ and G₂ are defined as in one of claims 1 and 4.
